# EUROPEAN PATENT APPLICATION

(11) **EP 3 173 401 A1**
(43) Date of publication of application: **31.05.2017**
(21) Application number: 15003404.9
(22) Date of filing: 27.11.2015
(51) Int. Cl.: C07C 245/20, C07C 249/06

(54) **PROCESS FOR THE SYNTHESIS OF ARYLDIAZONIUM SALTS USING NITROGEN OXIDES IN OXYGEN-CONTAINING GAS STREAMS, ESPECIALLY FROM INDUSTRIAL WASTE GASES**

(71) Applicant: Friedrich-Alexander-Universität Erlangen-Nürnberg, 91054 Erlangen (DE)
(72) Inventor: Hofmann, Dagmar, 91054 Erlangen (DE); Hofmann, Josefa, 91054 Erlangen (DE); Heinrich, Markus, 91094 Langensendelbach (DE)
(74) Representative: Forstmeyer, Dietmar

(57) **Abstract**

The present invention relates to a process for the synthesis of aryldiazonium salts using nitrogen oxides in oxygen-containing gas streams, especially from industrial waste gases.

## Description

The denitrification of industrial waste and flue gases is currently achieved through selective catalytic reduction (SCR) and selective non-catalytic reduction (SNCR). These techniques do however only produce nitrogen as product so that the synthetic potential of the nitrogen oxides, which are mainly nitrogen monoxide and nitrogen dioxide, remains unexploited. Only a few processes have so far been developed, which convert the nitrogen oxides from waste gas to cheap chemicals such as nitrate salts. The present invention is based on the task to combine denitrification with the synthesis of valuable chemical intermediates. A particularly valuable and broadly applicable group of compounds is that of aromatic diazonium salts.

Applications include radical reactions such as Sandmeyer, Meerwein and Gomberg-Bachmann reactions, transformations such as the Japp-Klingemann and the Balz-Schiemann reaction as well as numerous transition-metal catalyzed coupling reactions, for example including the Heck reaction. Most importantly, aromatic diazonium salts play a key role in the synthesis of a large variety of azo dyes, which represent ca. 60% of today's textile dyes.

In general, aromatic diazonium salts are prepared by diazotization of the corresponding aniline derivatives using nitrite salts in acidic media. The diazotization of anilines and acetanilides with nitrogen dioxide has been described previously, but highly concentrated nitrogen dioxide has so far been used for this purpose.

Moreover, such diazotizations with nitrogen dioxide have to date been carried out in the absence of air, which is entirely different to the composition of waste gases as they occur in industrial facilities or power plants.

The present invention relates to the diazotization of aromatic amines using a stream of low-concentrated nitrogen monoxide and/or nitrogen dioxide in air. The process is thus well suited for the denitrification of waste or flue gases.

The present invention provides a process for the preparation of a compound of formula (II) 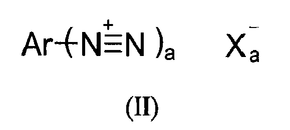 which is characterized in that a compound of formula (I) or a salt thereof

Ar(̵NHR')ₐ (I)

is reacted with nitrogen monoxide and/or nitrogen dioxide in the presence of oxygen,
wherein
Ar is an optionally substituted aryl or an optionally substituted heteroaryl group;
R' is hydrogen or a group of formula -CO-R", wherein R" is an optionally substituted alkyl, alkenyl, alkynyl, heteroalkyl, aryl, heteroaryl, cycloalkyl, alkylcycloalkyl, heteroalkylcycloalkyl, heterocycloalkyl, aralkyl or heteroaralkyl group;
X- is an anion; and
a is 1 or 2 (preferably 1).

Preferably, R" is a C₁₋₄ alkyl group or an optionally substituted phenyl group; especially a methyl group.

Especially preferably, R' is hydrogen.

Preferably, the anion X- is fluoride, chloride, bromide, sulfate (½ SO₄²⁻), hydrogensulfate, nitrate, nitrite, tetrafluoroborate, acetate, trifluoroacetate, hexafluorophosphate, hexafluoroantimonate, methanesulfonate, trifluoromethanesulfonate, the anion of an aromatic 1,2-dicarboximide or the anion of an aromatic 1,2-disulfonimide, toluene-4-sulfonate or monomethylsulfate. Preferably, the anion X- is derived from hydrochloric acid, hydrobromic acid, sulfuric acid, a hydrogensulfate salt, boric acid, tetrafluoroboric acid, methanesulfonic acid, acetic acid, trifluoroacetic acid, nitric acid or nitrous acid; especially preferably hydrochloric acid or sulfuric acid.

According to a preferred embodiment, the present invention relates to a process for preparing a compound of formula 2, 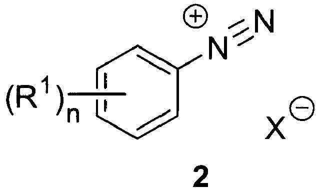 wherein a compound of formula 1 or a salt thereof 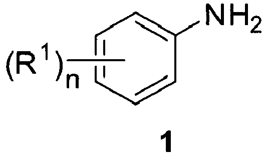 is reacted with nitrogen monoxide and/or nitrogen dioxide in the presence of oxygen,
wherein
n is 0,1,2,3,4 or 5;
R¹ is independently selected from a halogen atom, NO₂, N₃, OH, SH, NH₂, SO₃H, COOH or an optionally substituted alkyl, alkenyl, alkynyl, heteroalkyl, aryl, heteroaryl, cycloalkyl, alkylcycloalkyl, heteroalkylcycloalkyl, heterocycloalkyl, aralkyl or heteroaralkyl group; and
X- is defined as above.

According to a further preferred embodiment, the present invention relates to a process for preparing a compound of formula 4, 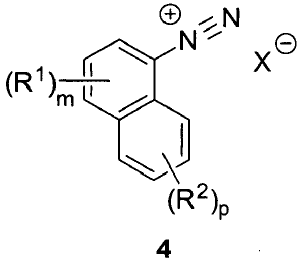 wherein a compound of formula 3 or a salt thereof 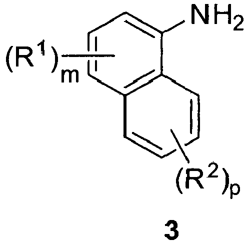 is reacted with nitrogen monoxide and/or nitrogen dioxide in the presence of oxygen,
wherein
m is 0,1,2 or 3;
p is 0,1,2,3 or 4;
R¹ is independently selected from a halogen atom, NO₂, N₃, OH, SH, NH₂, SO₃H, COOH or an optionally substituted alkyl, alkenyl, alkynyl, heteroalkyl, aryl, heteroaryl, cycloalkyl, alkylcycloalkyl, heteroalkylcycloalkyl, heterocycloalkyl, aralkyl or heteroaralkyl group;
R² is independently selected from a halogen atom, NO₂, N₃, OH, SH, NH₂, SO₃H, COOH or an optionally substituted alkyl, alkenyl, alkynyl, heteroalkyl, aryl, heteroaryl, cycloalkyl, alkylcycloalkyl, heteroalkylcycloalkyl, heterocycloalkyl, aralkyl or heteroaralkyl group; and
X- is defined as above.

According to a further preferred embodiment, the present invention relates to a process for preparing a compound of formula 6, 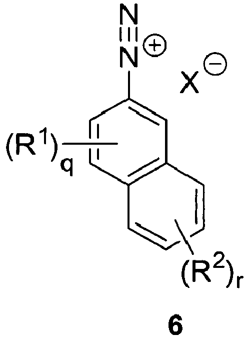 wherein a compound of formula 5 or a salt thereof 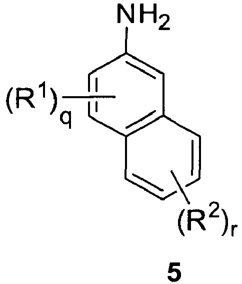 is reacted with nitrogen monoxide and/or nitrogen dioxide in the presence of oxygen,
wherein
q is 0,1,2 or 3;
r is 0,1,2,3 or 4;
R¹ is independently selected from a halogen atom, NO₂, N₃, OH, SH, NH₂, SO₃H, COOH or an optionally substituted alkyl, alkenyl, alkynyl, heteroalkyl, aryl, heteroaryl, cycloalkyl, alkylcycloalkyl, heteroalkylcycloalkyl, heterocycloalkyl, aralkyl or heteroaralkyl group;
R² is independently selected from a halogen atom, NO₂, N₃, OH, SH, NH₂, SO₃H, COOH or an optionally substituted alkyl, alkenyl, alkynyl, heteroalkyl, aryl, heteroaryl, cycloalkyl, alkylcycloalkyl, heteroalkylcycloalkyl, heterocycloalkyl, aralkyl or heteroaralkyl group; and
X- is defined as above.

Preferably, the compound of formula (I) is selected from the following compounds:

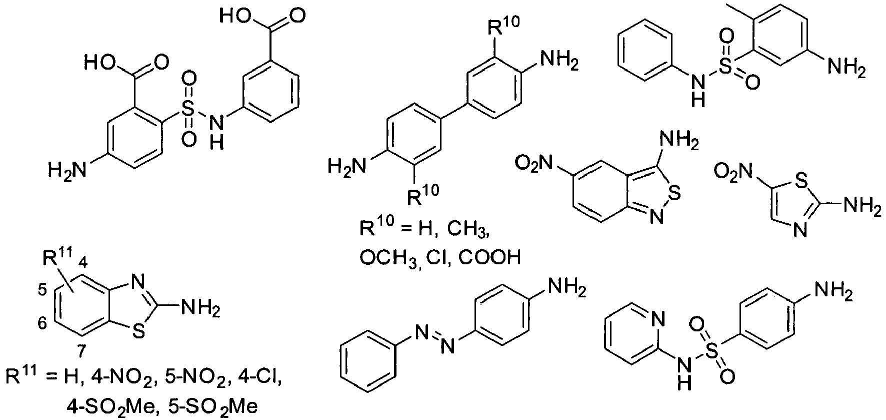

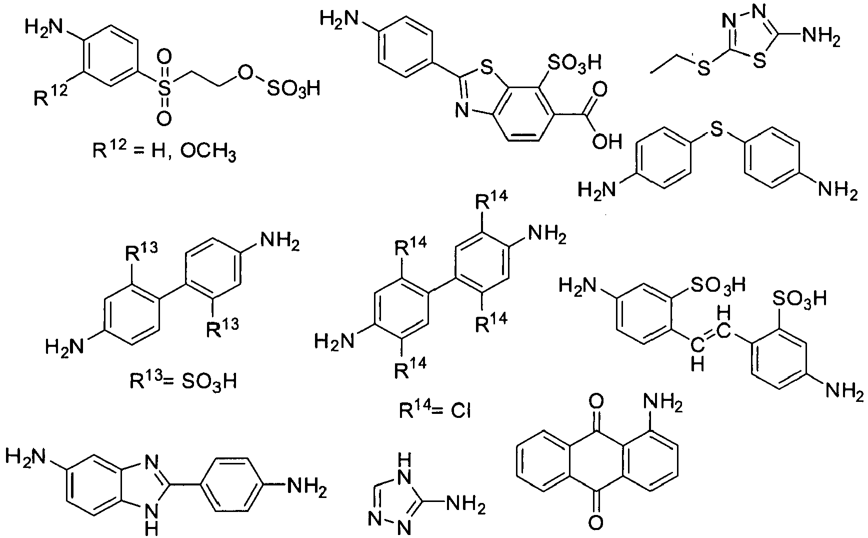

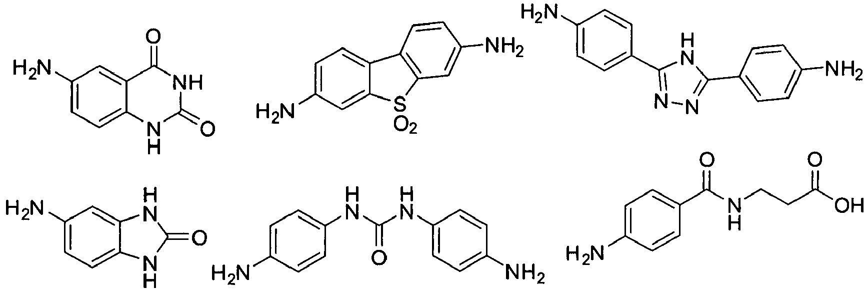

Compounds of formulas 1, 3 and 5 are preferred compounds of formula (I). Compounds of formulas 2, 4 and 6 are preferred compounds of formula (II).

Compounds of formula (I) as well as the preferred compounds of formulas 1, 3 and 5 may also be present in the form of their salts. Examples of salts of sufficiently basic compounds of formula (I), 1, 3 and 5 are salts of mineral acids like hydrochloric, hydrobromic, sulfuric and phosphoric acid; or salts of organic acids like methanesulfonic, p-toluenesulfonic, lactic, acetic, trifluoroacetic, citric, succinic, fumaric, maleic and salicylic acid. Further, a sufficiently acidic compound of formula (I), 1, 3 and 5 (e.g. a compound carrying a COOH or SO₃H group) may form alkali or earth alkali metal salts, for example sodium, potassium, lithium, caesium, calcium or magnesium salts; aluminum salts; ammonium salts; or organic base salts.

According to a further preferred embodiment, the process of the present invention further comprises a second step, in which the compound of formula (II) is reacted with a compound of formula Ar'-H to give a compound of formula Ar-N=N-Ar' wherein Ar is defined as above and Ar' is an optionally substituted aryl or an optionally substituted heteroaryl group.

Preferably, the reaction is performed at a pH of 9 or lower.

Further preferably, the reaction is performed in the presence of at least one solvent.

Especially preferably, the solvent is water or contains water.

Moreover preferably, the reaction is performed in water and at least one organic solvent.

Further preferably, the reaction is performed in the presence of water and at least one acid.

Especially preferably, the acid is selected from hydrochloric acid, hydrobromic acid, sulfuric acid, nitric acid, a hydrogensulfate salt, boric acid, tetrafluoroboric acid, methanesulfonic acid, acetic acid, trifluoroacetic acid; most preferably, the acid is hydrochloric acid or sulfuric acid.

Further preferably, the reaction is performed within a temperature range from -20° C to 150° C. This temperature range refers to the temperature of the reaction mixture or scrubber solution.

Moreover preferably, the reaction is performed within a pressure range from 0.1 to 10 bar.

Further preferably, the reaction is performed with a gas stream containing 0.001 - 50 vol.-% nitrogen monoxide.

Further preferably, the reaction is performed with a gas stream containing 0.001 - 50 vol.-% nitrogen dioxide.

Further preferably, the reaction is performed with a gas stream containing 0.01 - 50 vol.-% oxygen; preferably containing at least 1 vol.-% oxygen, more preferably at least 5 vol.-%; especially at least 10 vol.-%.

Especially preferably, the reaction is performed in the presence of water, at least one acid and at least one oxidant, which is preferably hydrogen peroxide.

The compounds of formula (II) can be used in various further reactions known to a person skilled in the art. It is for example possible to prepare biaryl compounds through a radical aryl-aryl coupling reaction or aryl-alkyl compounds through Meerwein arylation reactions. Moreover, compounds of formula (II) can be used for the preparation of halobenzenes or aromatic nitriles through Sandmeyer-type or Balz-Schiemann reactions. Compounds of formula (II) have furthermore been described as reactants in palladium-catalyzed cross-coupling reactions leading to biaryl compounds or arylvinyl compounds. Additional applications for compounds of formula (II) include the synthesis of diaryl azo compounds and aryl-heteroaryl azo compounds. Conversion of compounds of formula (II) into hydrazones through a Japp-Klingemann reaction or into aryl hydrazines via reduction has also been described. Compounds of formula (II) may further be used for the preparation of triazenes as well as for the functionalization and coating of surfaces and in nanotechnology applications. The expression halogen preferably refers to F, Cl, Br and I; more preferably to F, Cl or Br; especially to Cl or Br.

The expression alkyl refers to a saturated, straight-chain or branched hydrocarbon group that contains from 1 to 20 carbon atoms, preferably from 1 to 12 carbon atoms, especially from 1 to 6 (e.g. 1, 2, 3 or 4) carbon atoms, for example a methyl, ethyl, propyl, iso-propyl, n-butyl, iso-butyl, sec-butyl, tert-butyl, n-pentyl, iso-pentyl, n-hexyl, 2,2-dimethylbutyl or n-octyl group.

The expressions alkenyl and alkynyl refer to at least partially unsaturated, straight-chain or branched hydrocarbon groups that contain from 2 to 20 carbon atoms, preferably from 2 to 12 carbon atoms, especially from 2 to 6 (e.g. 2, 3 or 4) carbon atoms, for example an ethenyl (vinyl), propenyl (allyl), iso-propenyl, butenyl, ethinyl, propinyl, butinyl, acetylenyl, propargyl, isoprenyl or hex-2-enyl group. Preferably, alkenyl groups have one or two (especially preferably one) double bond(s), and alkynyl groups have one or two (especially preferably one) triple bond(s).

Furthermore, the terms alkyl, alkenyl and alkynyl refer to groups in which one or more hydrogen atoms have been replaced by a halogen atom (preferably F or Cl), e.g. a haloalkyl group, such as, for example, a 2,2,2-trichloroethyl or a trifluoromethyl group.

The expression heteroalkyl refers to an alkyl, alkenyl or alkynyl group in which one or more (preferably 1, 2 or 3) carbon atoms have been replaced by an oxygen, nitrogen, phosphorus, boron, selenium, silicon or sulfur atom (preferably by an oxygen, sulfur or nitrogen atom) or by a SO or a SO₂ group. The expression heteroalkyl furthermore refers to a carboxylic acid or to a group derived from a carboxylic acid, such as, for example, acyl, acylalkyl, alkoxycarbonyl, acyloxy, acyloxyalkyl, carboxyalkylamide or alkoxycarbonyloxy.

Preferably, a heteroalkyl group contains from 1 to 12 carbon atoms and from 1 to 4 hetero atoms selected from oxygen, nitrogen and sulphur (especially oxygen and nitrogen). Especially preferably, a heteroalkyl group contains from 1 to 6 (e.g. 1, 2, 3 or 4) carbon atoms and 1, 2 or 3 (especially 1 or 2) hetero atoms selected from oxygen, nitrogen and sulphur (especially oxygen and nitrogen). The term C₁-C₆ heteroalkyl refers to a heteroalkyl group containing from 1 to 6 carbon atoms and 1, 2 or 3 heteroatoms selected from O, S and/or N (especially O and/or N). The term C₁-C₄ heteroalkyl refers to a heteroalkyl group containing from 1 to 4 carbon atoms and 1, 2 or 3 heteroatoms selected from O, S and/or N (especially O and/or N). Furthermore, the term heteroalkyl refers to groups in which one or more hydrogen atoms have been replaced by a halogen atom (preferably F or Cl).

Examples of heteroalkyl groups are groups of formulae: R^{a}-O-Y^{a}-, R^{a}-S-Y^{a}-, R^{a}-SO-Y^{a}-, R^{a}-SO₂-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}-, R^{a}-N(R^{b})-CO-Y^{a}-, R^{a}-O-CO-N(R^{b})-Y^{a}-, R^{a}-N(R^{b})-CO-O-Y^{a}-, R^{a}-N(R^{b})-CO-N(R^{c})-Y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b})-Y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-, wherein R^{a} being a hydrogen atom, a C₁-C₆ alkyl, a C₂-C₆ alkenyl or a C₂-C₆ alkynyl group; R^{b} being a hydrogen atom, a C₁-C₆ alkyl, a C₂-C₆ alkenyl or a C₂-C₆ alkynyl group; R^{c} being a hydrogen atom, a C₁-C₆ alkyl, a C₂-C₆ alkenyl or a C₂-C₆ alkynyl group; R^{d} being a hydrogen atom, a C₁-C₆ alkyl, a C₂-C₆ alkenyl or a C₂-C₆ alkynyl group and Y^{a} being a bond, a C₁-C₆ alkylene, a C₂-C₆ alkenylene or a C₂-C₆ alkynylene group, wherein each heteroalkyl group contains at least one carbon atom and one or more hydrogen atoms may be replaced by fluorine or chlorine atoms.

Specific examples of heteroalkyl groups are methoxy, trifluoromethoxy, ethoxy, n-propyloxy, isopropyloxy, butoxy, *tert-*butyloxy, methoxymethyl, ethoxymethyl, -CH₂CH₂OH, -CH₂OH, -SO₂Me, -COOH, methoxyethyl, 1-methoxyethyl, 1-ethoxyethyl, 2-methoxyethyl or 2-ethoxyethyl, methylamino, ethylamino, propylamino, isopropylamino, dimethylamino, diethylamino, isopropylethylamino, methylamino methyl, ethylamino methyl, diisopropyl-amino ethyl, methylthio, ethylthio, isopropylthio, enol ether, dimethylamino methyl, dimethylamino ethyl, acetyl, propionyl, butyryloxy, acetyloxy, methoxycarbonyl, ethoxycarbonyl, propionyloxy, acetylamino or propionylamino, carboxymethyl, carboxyethyl or carboxypropyl, N-ethyl-N-methylcarbamoyl or N-methylcarbamoyl. Further examples of heteroalkyl groups are nitrile, isonitrile, cyanate, thiocyanate, isocyanate, isothiocyanate and alkylnitrile groups.

Further examples for heteroalkyl groups are hydroxyalkyl, alkoxy, haloalkoxy, alkylcarbonyl, haloalkylcarbonyl, alkylcarbonyloxy, haloalkylcarbonyloxy, alkoxycarbonyl, haloalkoxycarbonyl, alkylsulfonyl, haloalkylsulfonyl, alkylthio and haloalkylthio groups as well as groups of formula -NH(alkyl), -N(alkyl)₂, -NH(haloalkyl), -N(haloalkyl)₂, - N(alkyl)(haloalkyl), -NH(hydroxyalkyl), -N(hydroxyalkyl)₂, -NHCO(alkyl), - NHCO(haloalkyl), -NHSO₂(alkyl) and -NHSO₂(haloalkyl).

The expression cycloalkyl refers to a saturated or partially unsaturated (for example, a cycloalkenyl group) cyclic group that contains one or more rings (preferably 1 or 2), and contains from 3 to 14 ring carbon atoms, preferably from 3 to 10 (especially 3, 4, 5, 6 or 7) ring carbon atoms. The expression cycloalkyl refers furthermore to groups in which one or more hydrogen atoms have been replaced by fluorine, chlorine, bromine or iodine atoms or by OH, =O, SH, =S, -SO₃H, -SO₂NH₂, NH₂, =NH, N₃ or NO₂ groups, thus, for example, cyclic ketones such as, for example, cyclohexanone, 2-cyclohexenone or cyclopentanone. Further specific examples of cycloalkyl groups are a cyclopropyl, cyclobutyl, cyclopentyl, spiro[4,5]decanyl, norbornyl, cyclohexyl, cyclopentenyl, cyclohexadienyl, decalinyl, bicyclo-[4.3.0]nonyl, tetraline, cyclopentylcyclohexyl, fluorocyclohexyl or cyclohex-2-enyl group.

The expression heterocycloalkyl refers to a cycloalkyl group as defined above in which one or more (preferably 1, 2 or 3) ring carbon atoms have been replaced by an oxygen, nitrogen, silicon, selenium, phosphorus or sulfur atom (preferably by an oxygen, sulfur or nitrogen atom) or a SO group or a SO₂ group. A heterocycloalkyl group has preferably 1 or 2 ring(s) containing from 3 to 10 (especially 3, 4, 5, 6 or 7) ring atoms (preferably selected from C, O, N and S). The expression heterocycloalkyl refers furthermore to groups that are substituted by fluorine, chlorine, bromine or iodine atoms or by OH, =O, SH, =S, -SO₃H, -SO₂NH₂, NH₂, =NH, N₃ or NO₂ groups. Examples are a piperidyl, prolinyl, imidazolidinyl, piperazinyl, morpholinyl, urotropinyl, pyrrolidinyl, tetrahydrothiophenyl, tetrahydropyranyl, tetrahydrofuryl or 2-pyrazolinyl group and also lactames, lactones, cyclic imides and cyclic anhydrides.

The expression alkylcycloalkyl refers to groups that contain both cycloalkyl and also alkyl, alkenyl or alkynyl groups in accordance with the above definitions, for example alkylcycloalkyl, cycloalkylalkyl, alkylcycloalkenyl, alkenylcycloalkyl and alkynylcycloalkyl groups. An alkylcycloalkyl group preferably contains a cycloalkyl group that contains one or two rings having from 3 to 10 (especially 3, 4, 5, 6 or 7) ring carbon atoms, and one or two alkyl, alkenyl or alkynyl groups (especially alkyl groups) having 1 or 2 to 6 carbon atoms.

The expression heteroalkylcycloalkyl refers to alkylcycloalkyl groups as defined above in which one or more (preferably 1, 2 or 3) carbon atoms have been replaced by an oxygen, nitrogen, silicon, selenium, phosphorus or sulfur atom (preferably by an oxygen, sulfur or nitrogen atom) or a SO group or a SO₂ group. A heteroalkylcycloalkyl group preferably contains 1 or 2 rings having from 3 to 10 (especially 3, 4, 5, 6 or 7) ring atoms, and one or two alkyl, alkenyl, alkynyl or heteroalkyl groups (especially alkyl or heteroalkyl groups) having from 1 or 2 to 6 carbon atoms. Examples of such groups are alkylheterocycloalkyl, alkylheterocycloalkenyl, alkenylheterocycloalkyl, alkynylheterocycloalkyl, heteroalkylcycloalkyl, heteroalkylheterocycloalkyl and heteroalkylheterocycloalkenyl, the cyclic groups being saturated or mono-, di- or tri-unsaturated.

The expression aryl refers to an aromatic group that contains one or more rings containing from 6 to 14 ring carbon atoms, preferably from 6 to 10 (especially 6) ring carbon atoms. The expression aryl refers furthermore to groups that are substituted by fluorine, chlorine, bromine or iodine atoms or by OH, SH, SO₃H, SO₂NH₂, NH₂, N₃ or NO₂ groups. Examples are the phenyl, naphthyl, biphenyl, 2-fluorophenyl, anilinyl, 3-nitrophenyl or 4-hydroxyphenyl group.

The expression heteroaryl refers to an aromatic group that contains one or more rings containing from 5 to 14 ring atoms, preferably from 5 to 10 (especially 5 or 6 or 9 or 10) ring atoms, and contains one or more (preferably 1, 2, 3 or 4) oxygen, nitrogen, phosphorus or sulfur ring atoms (preferably O, S or N). The expression heteroaryl refers furthermore to groups that are substituted by fluorine, chlorine, bromine or iodine atoms or by OH, SH, SO₃H, SO₂NH₂, N₃, NH₂ or NO₂ groups. Examples are pyridyl (e.g. 4-pyridyl), imidazolyl (e.g. 2-imidazolyl), phenylpyrrolyl (e.g. 3-phenylpyrrolyl), thiazolyl, isothiazolyl, 1,2,3-triazolyl, 1,2,4-triazolyl, oxadiazolyl,thiadiazolyl, indolyl, indazolyl, tetrazolyl, pyrazinyl, pyrimidinyl, pyridazinyl, oxazolyl, isoxazolyl, triazolyl, tetrazolyl, isoxazolyl, indazolyl, indolyl, benzimidazolyl, benzoxazolyl, benzisoxazolyl, benzthiazolyl, pyridazinyl, quinolinyl, isoquinolinyl, pyrrolyl, purinyl, carbazolyl, acridinyl, pyrimidyl, 2,3'-bifuryl, pyrazolyl (e.g. 3-pyrazolyl) and isoquinolinyl groups.

The expression aralkyl refers to groups containing both aryl and also alkyl, alkenyl, alkynyl and/or cycloalkyl groups in accordance with the above definitions, such as, for example, aryl-alkyl, arylalkenyl, arylalkynyl, arylcycloalkyl, arylcycloalkenyl, alkylarylcycloalkyl and alkylarylcycloalkenyl groups. Specific examples of aralkyls are toluene, xylene, mesitylene, styrene, benzyl chloride, o-fluorotoluene, 1H-indene, tetraline, dihydronaphthalene, indanone, phenylcyclopentyl, cumene, cyclohexylphenyl, fluorene and indane. An aralkyl group preferably contains one or two aromatic ring systems (1 or 2 rings) containing from 6 to 10 carbon atoms and one or two alkyl, alkenyl and/or alkynyl groups containing from 1 or 2 to 6 carbon atoms and/or a cycloalkyl group containing 5 or 6 ring carbon atoms.

The expression heteroaralkyl refers to an aralkyl group as defined above in which one or more (preferably 1, 2, 3 or 4) carbon atoms have been replaced by an oxygen, nitrogen, silicon, selenium, phosphorus, boron or sulfur atom (preferably oxygen, sulfur or nitrogen), that is to say to groups containing both aryl or heteroaryl, respectively, and also alkyl, alkenyl, alkynyl and/or heteroalkyl and/or cycloalkyl and/or heterocycloalkyl groups in accordance with the above definitions. A heteroaralkyl group preferably contains one or two aromatic ring systems (1 or 2 rings) containing from 5 or 6 to 10 ring carbon atoms and one or two alkyl, alkenyl and/or alkynyl groups containing 1 or 2 to 6 carbon atoms and/or a cycloalkyl group containing 5 or 6 ring carbon atoms, wherein 1, 2, 3 or 4 of these carbon atoms have been replaced by oxygen, sulfur or nitrogen atoms.

Examples are arylheteroalkyl, arylheterocycloalkyl, arylheterocycloalkenyl, arylalkyl-heterocycloalkyl, arylalkenylheterocycloalkyl, arylalkynylheterocycloalkyl, arylalkylhetero-cycloalkenyl, heteroarylalkyl, heteroarylalkenyl, heteroarylalkynyl, heteroarylheteroalkyl, heteroarylcycloalkyl, heteroarylcycloalkenyl, heteroarylheterocycloalkyl, hetero-arylheterocycloalkenyl, heteroarylalkylcycloalkyl, heteroarylalkylheterocycloalkenyl, hetero-arylheteroalkylcycloalkyl, heteroarylheteroalkylcycloalkenyl and heteroarylheteroalkylhetero-cycloalkyl groups, the cyclic groups being saturated or mono-, di- or tri-unsaturated. Specific examples are a tetrahydroisoquinolinyl, benzoyl, 2- or 3-ethylindolyl, 4-methylpyridino, 2-, 3-or 4-methoxyphenyl, 4-ethoxyphenyl, 2-, 3- or 4-carboxyphenylalkyl group.

Further examples for heteroaralkyl groups are aryl, heteroaryl, arylsulfonyl, heteroarylsulfonyl, arylcarbonyl, heteroarylcarbonyl, aryloxy, aryloxycarbonyl, heteroaryloxy, heteroaryloxycarbonyl, arylcarbonyloxy, heteroarylcarbonyloxy, arylthio and heteroarylthio groups as well as groups of formula -NH(aryl), N(aryl)₂, -NH(heteroaryl), -N(heteroaryl)₂, -N(aryl)(heteroaryl), -N(aryl)(alkyl), -N(aryl)(haloalkyl), - N(heteroaryl)(alkyl), -N(hetero-aryl)(haloalkyl), -NHCO(aryl), -NHCO(heteroaryl), -NHSO₂(aryl), -NHSO₂(heteroaryl), -N₂(aryl) and -N₂(heteroaryl).

The term "haloalkyl", as used herein and in the haloalkyl units of haloalkoxy, describes straight-chain or branched alkyl groups having 1 to 10 carbon atoms (C₁-C₁₀-haloalkyl), preferably 1 to 4 carbon atoms (C₁-C₄-haloalkyl) and especially 1 to 2 carbon atoms (C₁-C₂-haloalkyl), where some or all of the hydrogen atoms in these groups are replaced by halogen atoms. Examples of C₁-C₂-haloalkyl are chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromomethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl. Examples of C₁-C₄-haloalkyl are, as well as those mentioned for C₁-C₂-haloalkyl, also 3,3,3-trifluoroprop-1-yl, 1,1,1-trifluoroprop-2-yl, 3,3,3-trichloroprop-1-yl, heptafluoroisopropyl, 1-chlorobutyl, 2-chlorobutyl, 3-chlorobutyl, 4-chlorobutyl, 1-fluorobutyl, 2-fluorobutyl, 3-fluorobutyl, 4-fluorobutyl and the like. Preference is given to fluoromethyl, 2-fluoroethyl and trifluoromethyl.

The term "hydroxyalkyl" denotes an alkyl group bearing one hydroxyl group, where the alkyl group optionally bears 1, 2 or 3 further substituents selected from halogen, alkyl, haloalkyl, cycloalkyl, alkoxy and haloalkoxy. Examples are -CH₂OH, -(CH₂)₂OH or -(CH₂)₃OH. The term "alkoxy" denotes straight-chain or branched saturated alkyl groups bonded via an oxygen atom and comprising 1 to 10 carbon atoms (C₁-C₁₀-alkoxy), preferably 1 to 4 carbon atoms (C₁-C₄-alkoxy), where the alkyl radical optionally bears 1, 2 or 3 substituents selected from cycloalkyl, alkoxy and haloalkoxy. Examples of C₁-C₄-alkoxy are methoxy, ethoxy, n-propoxy, 1-methylethoxy (isopropoxy), n-butoxy, 1-methylpropoxy (sec-butoxy), 2-methylpropoxy (isobutoxy) and 1,1-dimethyloxy (tert-butoxy). Examples of C₁-C₁₀-alkoxy are, as well as those mentioned for C₁-C₄-alkoxy, pentyloxy, hexyloxy and the like. Preference is given to methoxy, ethoxy, n-propoxy and -OCH₂-cyclo-pentyl.

The term "haloalkoxy" describes straight-chain or branched saturated haloalkyl groups bonded via an oxygen atom and comprising 1 to 10 carbon atoms (C₁-C₁₀-haloalkoxy), preferably 1 to 4 carbon atoms (C₁-C₄-haloalkoxy). Examples thereof are chloromethoxy, bromomethoxy, dichloromethoxy, trichloromethoxy, fluoromethoxy, difluoromethoxy, trifluoromethoxy, chlorofluoromethoxy, dichlorofluoromethoxy, chlorodifluoromethoxy, 1-chloroethoxy, 1-bromoethoxy, 1-fluoroethoxy, 2-fluoroethoxy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, 1,1,2,2-tetrafluoroethoxy, 1-chloro-1,2,2-trifluoroethoxy, pentafluoroethoxy, 3,3,3-trifluoroprop-1-oxy, 1,1,1-trifluoroprop-2-oxy, 3,3,3-trichloroprop-1-oxy, 1-chlorobutoxy, 2-chlorobutoxy, 3-chlorobutoxy, 4-chlorobutoxy, 1-fluorobutoxy, 2-fluorobutoxy, 3-fluorobutoxy, 4-fluorobutoxy and the like; preference is given to fluoromethoxy, difluoromethoxy and trifluoromethoxy.

The term "alkylcarbonyl" denotes alkyl radicals bonded via a carbonyl group and having 1 to 10 carbon atoms (C₁-C₁₀-alkylcarbonyl), where the alkyl radical optionally bears 1, 2 or 3 substituents selected from halogen, alkyl, haloalkyl, cycloalkyl, alkoxy and haloalkoxy. Examples thereof are methylcarbonyl (acetyl), ethylcarbonyl, propylcarbonyl, isopropylcarbonyl, n-butylcarbonyl, sec-butylcarbonyl, isobutylcarbonyl and tert-butylcarbonyl; preferably methylcarbonyl and ethylcarbonyl.

The term "haloalkylcarbonyl" denotes haloalkyl radicals bonded via a carbonyl group and having 1 to 10 carbon atoms (C₁-C₁₀-haloalkylcarbonyl). Examples thereof are fluoromethylcarbonyl, difluoromethylcarbonyl, trifluoromethylcarbonyl, 1-fluoroethylcarbonyl, 2-fluoroethylcarbonyl, 1,1-difluoroethylcarbonyl, 2,2-difluoroethylcarbonyl, 2,2,2-trifluoroethylcarbonyl, pentafluoroethylcarbonyl and the like; preference is given to fluoromethylcarbonyl, difluoromethylcarbonyl and trifluoromethylcarbonyl.

The term "alkylcarbonyloxy" denotes alkyl radicals bonded via a carbonyloxy group and having 1 to 10 carbon atoms (C₁-C₁₀-alkylcarbonyloxy), where the alkyl radical optionally bears 1, 2 or 3 substituents selected from cycloalkyl, alkoxy and haloalkoxy. Examples thereof are methylcarbonyloxy (acetoxy), ethylcarbonyloxy, propylcarbonyloxy and isopropylcarbonyloxy, preferably methylcarbonyloxy and ethylcarbonyloxy.

The term "haloalkylcarbonyloxy" denotes haloalkyl radicals bonded via a carbonyloxy group and having 1 to 10 carbon atoms (C₁-C₁₀-haloalkylcarbonyloxy), preferably 1 to 4 carbon atoms (C₁-C₄-haloalkylcarbonyloxy). Examples thereof are fluoromethylcarbonyloxy, difluoromethylcarbonyloxy, trifluoromethylcarbonyloxy, 1 -fluoroethylcarbonyloxy, 2-fluoroethylcarbonyloxy, 1,1-difluoroethylcarbonyloxy, 2,2-difluoroethylcarbonyloxy, 2,2,2-trifluoroethylcarbonyloxy, pentafluoroethylcarbonyloxy and the like; preference is given to fluoromethylcarbonyloxy, difluoromethylcarbonyloxy and trifluoromethylcarbonyloxy.

The term "alkoxycarbonyl" denotes alkoxy radicals bonded via a carbonyl group and having 1 to 10 carbon atoms (C₁-C₁₀-alkoxycarbonyl), preferably 1 to 4 carbon atoms (C₁-C₄-alkoxycarbonyl), where the alkyl radical optionally bears 1, 2 or 3 substituents selected from cycloalkyl, alkoxy and haloalkoxy. Examples thereof are methoxycarbonyl, ethoxycarbonyl, propoxycarbonyl, isopropoxycarbonyl, n-butoxycarbonyl, sec-butoxycarbonyl, isobutoxycarbonyl and tert-butoxycarbonyl; preference is given to methoxycarbonyl, ethoxycarbonyl, propoxycarbonyl and isopropoxycarbonyl.

The term "haloalkoxycarbonyl" denotes haloalkoxy radicals bonded via a carbonyl group and having 1 to 10 carbon atoms (C₁-C₁₀-haloalkoxycarbonyl), preferably 1 to 4 carbon atoms (C₁-C₄-haloalkoxycarbonyl). Examples thereof are fluoromethoxycarbonyl, difluoromethoxycarbonyl, trifluoromethoxycarbonyl, 1-fluoroethoxycarbonyl, 2-fluoroethoxycarbonyl, 1,1-difluoroethoxycarbonyl, 2,2-difluoroethoxycarbonyl, 2,2,2-trifluoroethoxycarbonyl, pentafluoroethoxycarbonyl and the like; preference is given to fluoromethoxycarbonyl, difluoromethoxycarbonyl and trifluoromethoxycarbonyl.

The term "alkylsulfonyl" denotes alkyl radicals bonded via a sulfonyl group (SO₂) and having 1 to 10 carbon atoms (C₁-C₁₀-alkylsulfonyl), preferably 1 to 4 carbon atoms (C₁-C₄-alkylsulfonyl), where the alkyl radical optionally bears 1, 2 or 3 substituents selected from cycloalkyl, alkoxy and haloalkoxy. Examples thereof are methylsulfonyl, ethylsulfonyl, propylsulfonyl, isopropylsulfonyl, n-butylsulfonyl, sec-butylsulfonyl, isobutylsulfonyl and tert-butylsulfonyl; preferably methylsulfonyl, ethylsulfonyl, propylsulfonyl and isopropylsulfonyl.

The term "haloalkylsulfonyl" denotes haloalkyl radicals bonded via a sulfonyl group (SO₂) and having 1 to 10 carbon atoms (C₁-C₁₀-haloalkylsulfonyl), preferably 1 to 4 carbon atoms (C₁-C₄-haloalkylsulfonyl). Examples thereof are fluoromethylsulfonyl, difluoromethylsulfonyl, trifluoromethylsulfonyl, 1-fluoroethylsulfonyl, 2-fluoroethylsulfonyl, 1,1-difluoroethylsulfonyl, 2,2-difluoroethylsulfonyl, 2,2,2-trifluoroethylsulfonyl, pentafluoroethylsulfonyl and the like; preferably fluoromethylsulfonyl, difluoromethylsulfonyl and trifluoromethylsulfonyl.

The term "arylsulfonyl" denotes aryl radicals bonded via a sulfonyl group (SO₂), where the aryl group optionally bears 1, 2, 3 or 4 substituents selected from halogen, alkyl, haloalkyl, alkoxy and haloalkoxy. Examples thereof are phenylsulfonyl, naphthylsulfonyl, fluorenylsulfonyl and the like; preference is given to phenylsulfonyl.

The term "heteroarylsulfonyl" denotes heteroaryl radicals bonded via a sulfonyl group (SO₂) and having 1 to 4 heteroatoms selected from O, N, S and SO₂, where the heteroaryl group optionally bears 1, 2, 3 or 4 substituents selected from halogen, alkyl, haloalkyl, alkoxy and haloalkoxy. Examples thereof are 2-pyrrolylsulfonyl, 2-furanylsulfonyl, 2-thienylsulfonyl, 3-pyrazolylsulfonyl, 2-imidazolylsulfonyl, 2-oxazolylsulfonyl, 4-pyridylsulfonyl and the like; preferably 2-pyrrolylsulfonyl, 2-furanylsulfonyl and 4-pyridylsulfonyl.

The term "arylcarbonyl" denotes aryl radicals bonded via a carbonyl group, where the aryl group optionally bears 1, 2, 3, 4 or 5 substituents selected from halogen, alkyl, haloalkyl, alkoxy or haloalkoxy. Examples thereof are phenylcarbonyl, 4-nitrophenylcarbonyl, 2-methoxyphenylcarbonyl, 4-chlorophenylcarbonyl, 2,4-dichlorophenylcarbonyl, 4-nitrophenylcarbonyl or naphthylcarbonyl, preferably phenylcarbonyl.

The term "heteroarylcarbonyl" denotes heteroaryl radicals bonded via a carbonyl group and having 1 to 4 heteroatoms selected from O, N, S and SO₂, where the heteroaryl group optionally bears 1, 2, 3 or 4 substituents selected from halogen, alkyl, haloalkyl, alkoxy and haloalkoxy. Examples thereof are 2-pyrrolylcarbonyl, 2-furanylcarbonyl, 2-thienylcarbonyl, 3-pyrazolylcarbonyl, 2-imidazolylcarbonyl, 2-oxazolylcarbonyl, 2-thiazolylcarbonyl, 4-triazolylcarbonyl, 4-pyridylcarbonyl and the like.

The term "aryloxy" denotes an aryl radical bonded via an oxygen atom, where the aryl group optionally bears 1, 2, 3, 4 or 5 substituents selected from halogen, alkyl, haloalkyl, alkoxy and haloalkoxy. Examples thereof are phenyloxy (phenoxy), naphthyloxy, fluorenyloxy and the like; preference is given to phenoxy.

The term "aryloxycarbonyl" denotes aryloxy radicals bonded via a carbonyl group and having 6 to 14 carbon atoms, where the aryl group optionally bears 1, 2, 3 or 4 substituents selected from halogen, alkyl, haloalkyl, alkoxy and haloalkoxy. Examples thereof are phenyloxycarbonyl (phenoxycarbonyl), naphthyloxycarbonyl, fluorenyloxycarbonyl and the like; preference is given to phenoxycarbonyl.

The term "heteroaryloxy" denotes heteroaryl radicals bonded via an oxygen atom and having 1 to 4 heteroatoms selected from O, N, S and SO₂, where the heteroaryl group optionally bears 1, 2, 3 or 4 substituents selected from halogen, alkyl, haloalkyl, alkoxy and haloalkoxy. Examples thereof are pyrrolyloxy, furanyloxy, thienyloxy, pyrazolyloxy, imidazolyloxy, oxazolyloxy, thiazolyloxy, pyridyloxy, pyrazinyloxy, pyridazinyloxy, pyrimidyloxy and the like; preferably pyrazolyloxy or pyridyloxy.

The term "heteroaryloxycarbonyl" denotes heteroaryloxy radicals bonded via a carbonyl group and having 1 to 4 heteroatoms selected from O, N, S and SO₂, where the heteroaryl group optionally bears 1, 2, 3 or 4 substituents selected from halogen, alkyl, haloalkyl, alkoxy and haloalkoxy. Examples thereof are pyrrolyloxycarbonyl, furanyloxycarbonyl, thienyloxycarbonyl, pyrazolyloxycarbonyl, imidazolyloxycarbonyl, oxazolyloxycarbonyl, thiazolyloxycarbonyl, pyridyloxycarbonyl, pyrazinyloxycarbonyl, pyridazinyloxycarbonyl, pyrimidyloxycarbonyl and the like; preferably imidazolyloxycarbonyl or oxazolyloxycarbonyl.

The term "arylcarbonyloxy" denotes aryl radicals bonded via a carbonyloxy group, where the aryl group optionally bears 1, 2, 3 or 4 substituents selected from halogen, alkyl, haloalkyl, alkoxy and haloalkoxy. Examples thereof are phenylcarbonyloxy, naphthylcarbonyloxy, fluorenylcarbonyloxy, anthracenylcarbonyloxy and the like; preferably phenylcarbonyloxy.

The term "heteroarylcarbonyloxy" denotes heteroaryl radicals bonded via a carbonyloxy group and having 1 to 4 heteroatoms selected from O, N, S and SO₂, where the heteroaryl group optionally bears 1, 2, 3 or 4 substituents selected from halogen, alkyl, haloalkyl, alkoxy and haloalkoxy. Examples thereof are 2-pyrrolylcarbonyloxy, 2-furanylcarbonyloxy, 2-thienylcarbonyloxy, 3-pyrazolylcarbonyloxy, 2-imidazolylcarbonyloxy, 2-oxazolylcarbonyloxy, 2-thiazolylcarbonyloxy, 4-triazolylcarbonyloxy, 4-pyridylcarbonyloxy and the like.

The term "alkylthio" denotes alkyl radicals bonded via a sulfur atom and having 1 to 10 carbon atoms (C₁-C₁₀-alkylthio), more preferably 1 to 6 carbon atoms (C₁-C₆-alkylthio), particularly 1 to 4 carbon atoms (C₁-C₄-alkylthio) and especially 1 to 3 carbon atoms (C₁-C₃-alkylthio), where the alkyl radical optionally bears 1, 2 or 3 substituents selected from halogen, alkyl, haloalkyl, cycloalkyl, alkoxy and haloalkoxy. Examples thereof are methylthio, ethylthio, n-propylthio, 1-methylethylthio (isopropylthio), n-butylthio, 1-methylpropylthio (sec-butylthio), 2-methylpropylthio (isobutylthio) and 1,1-dimethylethylthio (tert-butylthio) and the like; preferably methylthio, ethylthio and n-propylthio.

The term "haloalkylthio" describes haloalkyl groups bonded via a sulfur atom and having 1 to 10 carbon atoms (C₁-C₁₀-haloalkylthio), more preferably 1 to 6 carbon atoms (C₁-C₆-haloalkylthio), particularly 1 to 4 carbon atoms (C₁-C₄-haloalkylthio) and especially 1 to 3 carbon atoms (C₁-C₃-haloalkylthio). Examples thereof are chloromethylthio, bromomethylthio, dichloromethylthio, trichloromethylthio, fluoromethylthio, difluoromethylthio, trifluoromethylthio, chlorofluoromethylthio, dichlorofluoromethylthio, chlorodifluoromethylthio, 1-chloroethylthio, 1-bromoethylthio, 1-fluoroethylthio, 2-fluoroethylthio, 2,2-difluoroethylthio, 2,2,2-trifluoroethylthio, 2-chloro-2-fluoroethylthio, 2-chloro-2,2-difluoroethylthio, 2,2-dichloro-2-fluoroethylthio, 2,2,2-trichloroethylthio, 1,1,2,2-tetrafluoroethylthio, 1-chloro-1,2,2-trifluoroethylthio, pentafluoroethylthio, 3,3,3-trifluoroprop-1-ylthio, 1,1,1-trifluoroprop-2-ylthio, 3,3,3-trichloroprop-1-ylthio, 1-chlorobutylthio, 2-chlorobutylthio, 3-chlorobutylthio, 4-chlorobutylthio, 1-fluorobutylthio, 2-fluorobutylthio, 3-fluorobutylthio, 4-fluorobutylthio and the like; preference is given to fluoromethylthio, 2-fluoroethylthio and trifluoromethylthio.

The term "arylthio" denotes aryl radicals bonded via a sulfur atom, where the aryl group optionally bears 1, 2, 3 or 4 substituents selected from halogen, alkyl, haloalkyl, alkoxy and haloalkoxy. Examples thereof are phenylthio, naphthylthio, fluorenylthio and the like; preference is given to phenylthio.

The term "heteroarylthio" denotes heteroaryl radicals bonded via a sulfur atom and having 1 to 4 heteroatoms selected from O, N, S and SO₂, where the heteroaryl group optionally bears 1, 2, 3 or 4 substituents selected from halogen, alkyl, haloalkyl, alkoxy and haloalkoxy. Examples thereof are 2-pyrrolylthio, 3-furanylthio, 3-thienylthio, 2-pyridylthio and the like; preferably 2-pyridylthio and 4-pyridylthio.

As already stated above, the expressions cycloalkyl, heterocycloalkyl, alkylcycloalkyl, heteroalkylcycloalkyl, aryl, heteroaryl, aralkyl and heteroaralkyl also refer to groups that are substituted by fluorine, chlorine, bromine or iodine atoms or by OH, =O, SH, =S, -SO₃H, - SO₂NH₂, NH₂, =NH, N₃ or NO₂ groups.

The expression "optionally substituted" especially refers to groups that are optionally substituted by fluorine, chlorine, bromine or iodine atoms or by OH, =O, SH, =S, -SO₃H, - COOH, -SO₂NH₂, NH₂, =NH, N₃ or NO₂ groups. This expression refers furthermore to groups that may be substituted by one, two, three or more C₁-C₁₀ alkyl, C₂-C₁₀ alkenyl, C₂-C₁₀ alkynyl, C₁-C₁₀ heteroalkyl, C₃-C₁₈ cycloalkyl, C₂-C₁₇ heterocycloalkyl, C₄-C₂₀ alkylcyclo-alkyl, C₂-C₁₉ heteroalkylcycloalkyl, C₆-C₁₈ aryl, C₁-C₁₇ heteroaryl, C₇-C₂₀ aralkyl or C₂-C₁₉ heteroaralkyl groups. This expression refers furthermore especially to groups that may be substituted by one, two, three or more unsubstituted C₁-C₆ alkyl, C₂-C₆ alkenyl, C₂-C₆ alkynyl, C₁-C₆ heteroalkyl, C₃-C₁₀ cycloalkyl, C₂-C₉ heterocycloalkyl, C₇-C₁₂ alkylcycloalkyl, C₂-C₁₁ heteroalkylcycloalkyl, C₆-C₁₀ aryl, C₁-C₉ heteroaryl, C₇-C₁₂ aralkyl or C₂-C₁₁ heteroaralkyl groups.

According to a preferred embodiment, all alkyl, alkenyl, alkynyl, heteroalkyl, aryl, heteroaryl, cycloalkyl, heterocycloalkyl, alkylcycloalkyl, heteroalkylcycloalkyl, aralkyl and heteroaralkyl groups described herein may optionally be substituted.

When an aryl, heteroaryl, cycloalkyl, alkylcycloalkyl, heteroalkylcycloalkyl, heterocycloalkyl, aralkyl or heteroaralkyl group contains more than one ring, these rings may be bonded to each other via a single or double bond or these rings may be annulated.

The details given hereinafter regarding preferred configurations of the processes according to the invention, especially regarding preferred configurations of the reactants and products and the reaction conditions of the processes according to the invention, apply either taken alone or, more particularly, in any conceivable combination with one another.

The reactions described herein are preferably performed in reaction vessels customary for such reactions, and the reaction regime can be configured in a continuous, semicontinuous or batchwise manner.

In general, the respective reactions will be performed under atmospheric pressure. The reactions can, however, also be performed under reduced (e.g. 0.1 to 1.0 bar) or elevated pressure (e.g. >1.0 to 10 bar).

More particularly, it is preferable to combine the embodiments with one another in any desired combination.

According to a preferred embodiment, Ar is an optionally substituted phenyl group, an optionally substituted naphthyl group or an optionally substituted biphenyl group.

In the context of the present invention, n is preferably 0, 1, 2, 3 or 4, especially 0, 1, 2 or 3, more preferably 1 or 2.

In the context of the present invention, m is preferably 0, 1 or 2, especially 0 or 1, more preferably 1.

In the context of the present invention, q is preferably 0, 1 or 2, especially 0 or 1, more preferably 1.

In the context of the present invention, p is preferably 0, 1, 2 or 3, especially 0, 1 or 2, more preferably 0 or 1.

In the context of the present invention, r is preferably 0, 1, 2 or 3, especially 0, 1 or 2, more preferably 0 or 1.

In the context of the present invention, R¹ is preferably independently halogen, hydroxy, amino, alkyl, alkoxy, nitro, cyano, aryl, heteroaryl, NHCOCH₃, arylazo (such as e.g. phenylazo), heteroarylazo, COOH, SO₃H, SO₃M, or COOM, wherein M is Li, Na, K, Cs, Mg, Ca, Al or NH₄⁺. More preferably, R¹ is independently halogen, alkyl, alkoxy, hydroxy, amino, nitro, cyano, SO₃H or COOH.

In the context of the present invention, R² is preferably independently halogen, hydroxy, amino, alkyl, alkoxy, nitro, cyano, aryl, heteroaryl, NHCOCH₃, arylazo (such as e.g. phenylazo), heteroarylazo, COOH, SO₃H, SO₃M, or COOM. More preferably, R² is independently halogen, alkyl, alkoxy, hydroxy, amino, nitro, cyano, SO₃H or COOH.

More particularly, for compounds of structure 1 and 2, R¹ is preferably independently 2-Cl, 4-SO₂CH₃, 4-NHCOCH₃, 2-SO₃H, 2-OH, 4-SO₂NH₂, 4-NO₂, 3-SO₃H, 2-Br, 2-NO₂, 4-OH, 3-COOH, 4-SO₃H, 2-NH₂, 3-OH, 3-Cl, 2-CN, 4-Cl, 4-CH₃, 3-NH₂, 4-NH₂ or 3-NO₂.

The stated positions for compounds of formulas 1 and 2 are based on the 1 position via which the aryl radical which derives from the compound of the formula 1 or 2 is bonded to the diazonium or amine moiety.

More particularly, for compounds of structure 3 and 4, R¹ is 2-SO₃H, 3-SO₃H, 4-SO₃H, 2-COOH, 3-COOH, 4-COOH, 2-OH, 3-OH and 4-OH.

More particularly, for compounds of structure 3 and 4, R² is 5-SO₃H, 6-SO₃H, 7-SO₃H, 8-SO₃H, 5-Cl, 6-Cl, 7-Cl, 8-Cl, 5-OMe, 6-OMe, 7-OMe, 8-OMe and 5-NH₂.

More particularly, for compounds of structure 5 and 6, R¹ is Prefereably 1-COOH, 3-COOH, 4-COOH, 1-SO₃H, 3-SO₃H, 4-SO₃H, 1-OH, 3-OH and 4-OH.

More particularly, for compounds of structure 5 and 6, R² is 5-SO₃H, 6-SO₃H, 7-SO₃H, 8-SO₃H, 5-Cl, 6-Cl, 7-Cl, 8-Cl, 5-OMe, 6-OMe, 7-OMe and 8-OMe.

The stated positions for compounds of formulas 3, 4, 5 and 6 are based on numbering shown below.

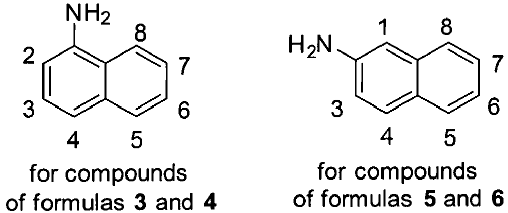

In the context of the present invention, X⁻ is preferably a halide, such as fluoride, chloride, bromide, iodide or BF₄⁻, PF₆⁻, hydrogensulfate, sulfate (½ SO₄²⁻), acetate, the anion of an aromatic 1,2-dicarboximide or the anion of an aromatic 1,2-disulfonimide. In the latter two cases, the anion forms through abstraction of the proton on the imide nitrogen atom. More preferably X⁻ is a halide, such as chloride or bromide, or BF₄⁻ or sulfate (½ SO₄²⁻).

The process of the present invention preferably combines the denitrification of waste gases, as they occur from industrial processes, from waste incineration or from power plants, with the synthesis of valuable and versatile intermediates.

According to a preferred embodiment of the present invention, the process of the present invention may be used for the denitrification of waste gases, especially industrially produced waste gases containing nitrogen monoxide and/or nitrogen dioxide and preferably containing at least 1% (by volume) of O₂ (especially at least 5%; most preferably at least 10% O₂).

Preferably, the nitrogen monoxide and/or nitrogen dioxide as well as the oxygen which are used in the process of the present invention are used in the form of waste gases.

For waste incineration, typical contents of the stream of waste gas are CO: 30 mg/m³; HCl: 1.500 mg/m³; HF: 14 mg/m³; SOₓ: 650 mg/m³ (reported as SO₂); NOₓ: 400 mg/Nm³ (reported as NO₂) and fly ash: 2.500 mg/Nm³. [Publication No. 4196 by the German Bundesumweltamt (available online via www.umweltbundesamt.de/sites/default/files/medien/ 461/publikationen/4196.pdf or http://www.uba.de/uba-info-medien/4196.html, 10.10.2015)]

NOₓ hereby corresponds to the combined amounts of the two most important nitrogen oxides occuring in waste gases, which are nitrogen monoxide (NO) and nitrogen dioxide (NO₂).

Other nitrogen oxides such as N₂O₄, N₂O, N₂O₃, N₂O₅ and N₄O may also be present in the gas stream or gas mixture used for the process. Within this description, the process is described for nitrogen monoxide and nitrogen dioxide as the two major representative components. Nitrogen dioxide can hereby be partially present in the form of its dimer N₂O₄ or in the form of its adduct with NO, which is N₂O₃. As the compounds N₂O₃ and N₂O₄ are part of a dynamic equilibrium with their corresponding monomers NO and NO₂, the process can also be used for the removal of N₂O₃ and N₂O₄ from gas streams of gas mixtures.

In some industrial fields such as metal processing and etching, for which concentrated nitric acid is commonly used, waste gases with NOₓ contents of up to 20 vol.-% are produced. A major difficulty in denitrification of such highly concentrated gas streams often is that the concentrations of NOₓ varies rapidly over time. Thus, the denitrification system has to be able to respond quickly to changes in the NOₓ concentration to achieve a high degree of purification at all times.

A further industrial process leading to gas streams containing unsteady concentrations of NOₓ is the production of nitric acid.

Especially for gas streams containing unsteady concentrations of NOₓ, wet scrubbers represent a valuable alternative to the denitrification methods SCR and SNCR. A typical scrubber solution used for such purpose is aqueous hydrogen peroxide (H₂O₂). [Curtius, F.; Oxidative washing process for cleaning waste gases. EP 0487834 (A1*)*; Chem. Abstr. 115:189023, 1991]

The main task of hydrogen peroxide within the denitrification is to counterbalance the low solubility of nitrogen monoxide in water or aqueous solutions through rapid oxidation to nitrogen dioxide. In this way, nitrogen monoxide is continuously removed from the unfavorable gas-liquid equilibrium so that further nitrogen monoxide can be absorbed. Nitrogen dioxide, in contrast, is well soluble in water and is thus easily removed from the stream of waste gas. Subsequently, NO₂ undergoes disportionation to nitric acid (HNO₃) and nitrous acid (HNO₂), of which the latter is again oxidized by hydrogen peroxide to give nitric acid. Nitric acid or nitrate salts are thus obtained as final products from such denitrification process.

In general, the ratio of NO and NO₂ in the waste gas depends on the temperature and on the amount of oxygen. High temperatures and low concentrations of oxygen favor NO. Due to the underlying equilibrium gas streams containing oxygen, as it is typically the case for waste gases, such gases always contain nitrogen monoxide and nitrogen dioxide. Within the description of this invention the existence of both compounds, nitrogen monoxide and nitrogen dioxoide, in the oxygen-containing gas stream is thus assumed.

The compounds of formula (II), which are obtained from the process, can be employed as starting materials in various further reactions. Preferably, these follow-up reactions are carried out directly with the solution obtained from the batch reactor or from the wet scrubber after denitrification without intermediate work-up. Possible applications are mentioned in the introductory section. In the examples section, particular examples are given for applications in radical aryl-aryl [A. Wetzel, V. Ehrhardt, M. R. Heinrich, Angew. Chem. Int. Ed. 2008, 47, 9130-9133.] and aryl-heteroaryl coupling reactions [A. Wetzel, G. Pratsch, R. Kolb, M. R. Heinrich, Chem. Eur. J. 2010, 16, 2547-2556.], Sandmeyer reactions [E. Müller, In Methoden der Organischen Chemie (Houben-Weyl), Georg Thieme Verlag, Stuttgart, Germany, 1960**.**], azo coupling leading to azo dyes (c.f. III.4.1) [K. Schwetlick, et al. In Organikum, VEB Deutscher Verlag der Wissenschaften, Berlin, Germany, 1971.] and Japp-Klingemann reactions [V. S. Matiychuk, M. A. Potopnyk, M. D. Obushak, J. Heterocyclic Chem. 2013, 50, E43.].

The products accessible through radical aryl-aryl coupling (c.f. III.2.1) are useful as hair colorants. [Braun, H.-J.; Chassot, L.; WO 9959527 (A2)*,* Chem. Abstr. 132:6216, 1999]

The products from Sandmeyer reactions (c.f. III.3.1) can be used as starting materials for various transition-metal catalyzed coupling reactions. [Modern Arylation Methods, Ackermann, L., Ed.; Wiley-VCH, Weinheim, 2009]

The products from Japp-Klingemann reactions (c.f. III.5.1) can be used as starting materials for heterocycles including indoles.

The process described herein allows the removal of NO and NO₂ from a gas stream with a scrubber solution containing compounds of formula (I) in water or mixtures of water and other solvents.

Preferably, the scrubber solution contains a compound of the formula (I) in water and an acid. Preferred acids are described below.

The denitrification process described in the present invention can be conducted as a batch process or as a continuous flow process.

A preferred setup for a batch process comprises a closed reaction vessel with pressure equilibration containing the compound of formula (I) in water or mixtures of water and other solvents. The stirred reaction mixture hereby typically occupies less than 5% of the total volume of the reaction vessel. The gas containing mainly NO and NO₂, and possibly amounts of other nitrogen oxides as described above, in air is then introduced in the reaction vessel. After completion of the denitrification of the gas introduced in the vessel, the reaction mixture containing the compound of formula (II) can be used for the purposes described above and in the examples section. The ratio of the compound of formula (II) to unreacted compound of formula (I) depends on the amounts of NO and NO₂ introduced into the reaction vessel.

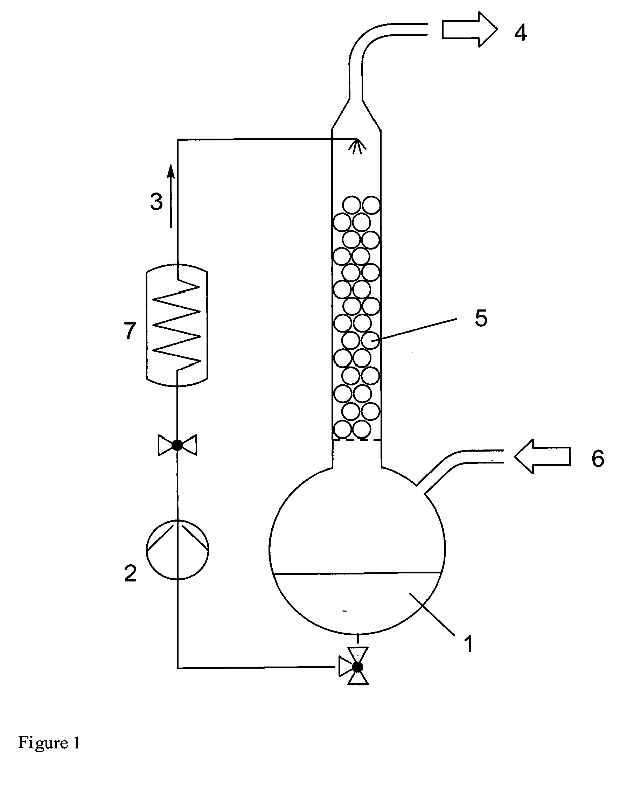

A preferred setup for a continuous process is a wet scrubber (Figure 1). In the scrubber the gas stream containing NO and NO₂, and which can additionally contain fly ashes, SO₂ and/or HCl, gets into contact with the scrubber solution over a column filled with suitable filling materials such as Raschig rings. Similar to the batch reaction, the scrubber solution contains the compound of formula (I) in water or mixtures of water with other solvents. After completion, the scrubber solution, which now contains the compound of formula (II) and, depending of the amounts of NO and NO₂ introduced, also the unreacted compound of formula (I) can be used for the purposes described above and in the examples section.

Figure 1 shows a scrubber for continuous flow denitrification. Therein, 1 denotes the scrubber solution, 2 denotes a pump, 3 denotes the flow of the scrubber solution, 4 denotes the purified gas stream, 5 denotes the column filled with Raschig rings, 6 denotes the gas stream/waste gas containing NO and/or NO₂ and 7 denotes a cooling or heating unit.

Typical scrubber units used in industry are of cylindrical shape and have dimensions of 5-15 m in length and 1-3.5 m in diameter. Such scrubbers are suitable for the denitrification of gas streams with flow rates in the range of 0.5-10 m³/second.

According to a further preferred embodiment, denitrification can be achieved by bubbling a gas stream containing NO and/or NO₂ through a solution of the compound of formula (I) in water or a mixture of water and another solvent.

The process for the conversion of compounds of formula (I) to compounds of formula (II) is preferably carried out with a gas stream containing a mixture of nitrogen monoxide and nitrogen dioxide. As described above, these two nitrogen oxides can interconvert via an equilibrium which depends on factors such as temperature and the concentration of oxygen or other suitable oxidants. For the process, gas streams with concentrations of nitrogen monoxide and nitrogen dioxide, each independently in the range of 0.001 - 50 vol.-%, may preferably be used.

Preferably, the conditions regarding temperature and the content of oxygen are chosen in a way that the equilibrium between nitrogen monoxide and nitrogen dioxide is shifted towards nitrogen dioxide. In particular, this is the case when the process is conducted at room temperature, or more generally at temperature below 100 °C.

To achieve a more rapid equilibration of the components nitrogen monoxide and nitrogen dioxide, the gas stream can optionally be brought into contact with suitable catalysts. As the process is preferably carried out in the presence of air, it can be assumed that due to the existing equilibrium between NO and NO₂, a mixture of these two compounds is present at all times in the gas stream or the gas mixture used for the process.

Suitable conditions are, for example, if a gas stream containing nitrogen dioxide and nitrogen dioxide in air, is used with an concentration of oxygen in the range of 10-20 vol.-%. Regarding other components of the gas stream besides nitrogen monoxide, nitrogen dioxide and oxygen, the preferred component is dinitrogen, as it is the major component in air.

Preferred conditions are also, if a gas stream from a waste incineration process is used. Such gas streams typically contain nitrogen monoxide and nitrogen dioxide (NO + NO₂: 400 mg/Nm³), as well as oxygen (6-10 vol.-%) and dinitrogen (88-94 vol.-%) as major components.

Currently, the denitrification of waste gases resulting from waste incineration is commonly (ca. 67% of waste incineration sites) achieved with the SCR technique, however comprising the mentioned drawbacks.

The importance of denitrification techniques can be derived from the NOₓ emissions reported for Germany in 2013, which amount to 300.000 tons for power plants, to 200.000 tons for industry and production and to ca. 15.000 tons for waste incineration.

Within the industrial sectors, 59% of the NOₓ emissions arise from incineration processes.

Beneficially, the preferred temperature range of 5 - 50 °C of the process fits well to a tail-end application of the process with the aim of denitrification of a stream of waste gas. Waste gases from industrial manufacturing, from power plants or from waste incineration are commonly purified by in a number of steps containing filters for fly ash, absorbers for sulfur oxides and scrubbers for acidic components. A "tail-end" application hereby means that the denitrification step is placed at the end of the purification sequence.

Advantages of tail-end denitrification usually are that the gas stream contains only low amounts of fly ash as well as only low concentrations of sulfur dioxide. For common techniques such as SCR and SNCR, the low temperature of the gas stream at the end of the sequence is however a significant drawback, which often requires reheating.

The process described in this invention, in contrast, is well suited for a tail-end application since a gas stream at low temperature will have a higher content of nitrogen dioxide relative to nitrogen monoxide. This is preferable for the process described herein.

Applications other than tail-end denitrification are however also possible. The process is, for example, not significantly perturbed by the presence of sulfur dioxide in the gas stream.

The process also tolerates the presence of acids such as hydrochloric acid in the gas stream. Moreover, the process is tolerant to the presence of fly ash in the gas stream or gas mixture employed.

The process for the conversion of compounds of formula (I) to compounds of formula (II) is preferably carried out in water as solvent.

Suitable solvents for the process of the present invention are aqueous solvents and organic solvents. Suitable organic solvents are, for example, short-chain nitriles, such as acetonitrile or propionitrile, amides such as N,N-dimethylformamide or N,N-dimethylacetamide, short-chain mono- or polyhydric alcohols such as methanol, ethanol, propanol, ethylene glycol or trifluoroethanol, dimethyl sulfoxide, open-chain and cyclic ethers such as diethyl ether, dioxane or tetrahydrofuran, sulfur compounds such as carbon disulfide or sulfolane, nitro compounds such as nitromethane, or mixtures of these organic solvents with one another. Preferred organic solvents are short-chain nitriles such as acetonitrile or propionitrile, amides such as N,N-dimethylformamide or N,N-dimethylacetamide, short-chain mono- or polyhydric alcohols such as methanol, ethanol, propanol, ethylene glycol or trifluoroethanol, dimethyl sulfoxide and mixtures of these solvents. Particular preference is given to acetonitrile.

In the case that one or more organic solvents are used, it is preferable to conduct the reaction in a mixture of water and the organic solvent or solvent mixture. The volume ratio of the organic solvent or the organic solvent mixture solvents to water can be chosen freely from 100/0 to 0/100.

Overall, the solvents or solvent mixtures used are preferably water or mixtures of the above-mentioned organic, water-miscible solvents or solvent mixtures of water and the aqueous acids mentioned.

As an alternative, not the pure solvents but mixtures which combine the solvent properties are used, or solubilizers are employed.

The term "solubilizer" denotes (interface-active) substances which, through their presence, make other compounds which are virtually insoluble in a solvent or solvent system soluble or emulsifiable in this solvent or solvent system, whether by entering into a molecular compound with the sparingly soluble substance or acting through micelle formation.

In a particularly preferred embodiment, aqueous solvents are used. Aqueous solvents are water or mixtures of water and at least one further solvent other than water. Solvents other than water are preferably organic solvents. Preferred organic solvents are water-miscible. Examples of water-miscible organic solvents are short-chain nitriles such as acetonitrile or propionitrile, amides such as N,N-dimethylformamide or N,N-dimethylacetamide, short-chain mono- or polyhydric alcohols such as methanol, ethanol, propanol, ethylene glycol or trifluoroethanol, and dimethyl sulfoxide. Particular preference is given to acetonitrile. Accordingly, particularly preferred solvents are water and aqueous solvents which, as well as water, comprise acetonitrile, i.e. water and water/acetonitrile mixtures.

Aqueous solvents which, as well as water, comprise at least one further solvent other than water comprise preferably 5 to 95% by weight, more preferably 10 to 95% by weight, even more preferably 20 to 95% by weight, yet more preferably and especially 30 to 95% by weight, and especially 40 to 95% by weight, of water, e.g. 50 to 90 or 60 to 90 or 70 to 90 or 75 to 85% by weight of water. The residual content corresponds to the further solvent(s).

The pH can be determined by means of customary methods, for example by means of indicators or standard pH meters, for example with glass electrodes or hydrogen electrodes, or with field-effect transistors. Typically, however, the pH is determined in a simple manner via the concentration of the base used, without taking activities into account.

Stated pH values are typically based on aqueous media, i.e. on the concentration/activity of an acid or base in water. If the reaction medium in which the reaction of the compound of formula (I) takes place is aqueous, the pH values are determined in the generally customary manner. If the reaction medium, in contrast, is nonaqueous, "within the acid range" in the context of the present invention means that the reaction medium in question comprises one or more acids in such a concentration that a purely aqueous medium (i.e. with water as the sole solvent) which comprised the same acid(s) in the same concentration would be acidic and would preferably have a pH of at most 7 (e.g. 7 to 0 or lower, e.g. to -1 or to -2), more preferably at most 5 (e.g. 5 to 0 or lower, e.g. to -1 or to -2), even more preferably at most 3 (e.g. 3 to 0 or lower, e.g. to -1 or to -2).

Further, the process of the present invention can also be conducted in a pH range of 7 - 9.

The above mentioned pH values refer to pH values that are obtained after the addition of at least one acid to the compound of formula (I) in at least one solvent. Suitable acids are described below.

In the context of the processes according to the invention, suitable acids are, for example, inorganic acids such as hydrochloric acid, hydrobromic acid, sulfuric acid or tetrafluoroboric acid. Also suitable in principle are organic acids, such as carboxylic acids (e.g. acetic acid, trifluoroacetic, benzoic acid) and sulfonic acids (e.g. methanesulfonic acid, trifluoromethanesulfonic acid, benzenesulfonic acid, p-toluenesulfonic acid).

Preference is given to the inorganic acids mentioned, among which preference is given to hydrochloric and sulfuric acid, preferably in the form of the aqueous solution thereof.

Related to this, the process is preferably carried out with a solution containing the compound of formula (I) in a mixture of water and an aqueous solution of one or more of the above mentioned acids.

Alternatively and additionally, the process can be carried out with a solution containing more than one the compounds of formula (I) in a mixture of water and an aqueous solution of one or more of the above mentioned acids.

For the process, it is preferable to use a gas stream in which the ratio of nitrogen dioxide and nitrogen monoxide is shifted towards nitrogen dioxide, which can for example by achieved by lowering the temperature.

For gas streams at higher temperatures, e.g. above 50 °C, it is preferable to add an oxidant to the solution containing the compound of formula (I). A preferred oxidant is hydrogen peroxide.

Alternatively, or additionally, for the case that gas streams at higher temperatures, e.g. above 50 °C, are used, the reaction solution can be cooled. This can be achieved by a cooling jacket for the batch setup or by passing the circulating scrubber solution through a cooling device in the continuous wet scrubber setup (see Figure 1, 7).

The process is especially preferably conducted with a high content of oxygen in the gas stream with a value above 15 vol.-%. Such a high content of oxygen is for example present in air, which contains approximately 20 vol.-% of oxygen. The process is nevertheless also suited for gas streams containing lower amounts of oxygen with values lower than 15 vol.-%. Typical oxygen contents in gas streams arising from waste incineration or from power plants are, for example, in the range of 5-15 vol.-%.

The process can be conducted at temperature of the reaction solution in the range of -20 to 150 °C, preferably 0 to 100 °C, more preferably 5 to 60 °C, even more preferably 10 to 35 °C.

The process, in which a compound of formula (I) is converted to a compound of formula (II) is preferably conducted in at least one solvent. The concentration of the compound of formula (I) in the reaction or scrubber solution can be chosen in the range of 0.001 to 10 mol/l, preferably in the range of 0.01 to 1 mol/l, more preferably in the range of 0.01 to 0.1 mol/l. Alternatively, the process can be conducted with a mixture of two or more compounds of formula (I), which are then converted to a mixture of the corresponding compounds of formula (II). The preferred concentration of each compound of formula (I) is as described above.

By means of the process of the present invention, the NO₂ content in streams of air can be reduced from values of 5000-60000 ppm to values below 200 ppm. This demonstrates the applicability of the process for the purpose of denitrification of waste gases.

### EXAMPLES

Solvents and reagents were used as received. ¹H-NMR spectra were recorded on 360 and 600 MHz spectrometers using CDCl₃ and DMSO-*d*₆ as solvents referenced to TMS (0 ppm), CHCl₃ (7.26 ppm) or DMSO-*d*₆ (2.50 ppm). Chemical shifts are reported in parts per million (ppm). Coupling constants are in Hertz (J Hz). The following abbreviations are used for the description of signals: s (singlet), d (doublet), dd (double doublet), t (triplet), q (quadruplet), m (multiplet). ¹³C-NMR spectra were recorded at 91 and 151 MHz in CDCl₃ using CDCl₃ (77.0 ppm) as standard. Mass spectra were recorded using electron impact (EI) or electron spray ionisation (ESI). Analytical TLC was carried out on Merck silica gel plates using short wave (254 nm) UV light to visualize components. Silica gel (Kieselgel 60, 40-63 µm) was used for flash column chromatography.

### Abbreviations:

EtOAc: ethyl acetate
THF: tetrahydrofuran
DMSO: dimethylsulfoxide

### I. General Methods for Denitrification combined with Diazotiation

### 1.1 Preparation of 4-Chlorophenyldiazonium Chloride in round bottom flask (batch reaction, GM 1)

In a 250 mL round bottom flask equipped with a balloon for pressure equilibration, nitrogen monoxide (0.10-4.00 mmol) was added by syringe to a mixture of 4-chloroaniline (**1a**) (1.00 mmol, 128 mg) in a mixture of water and 1 N HCl (3:1 v/v, 16 ml). After vigorous stirring at room temperature, the reaction mixture was concentrated under reduced pressure and analyzed by ¹H-NMR spectroscopy using maleic acid as internal standard. The results obtained under these conditions are summarized in the table below.

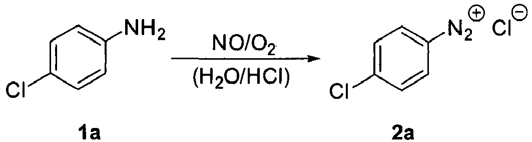

| Entry | NO (equiv) | Reaction time (min) | Diazotization (%) [max. %] | Percentage of NO inc. in **2a** |
|---|---|---|---|---|
| 1 | 4.0 | 120 | 100 [100] | - |
| 2 | 3.0 | 120 | 100 [100] | - |
| 3 | 2.3 | 120 | 100 [100] | - |
| 4 | 2.0 | 120 | 100 [100] | 50 |
| 5 | 2.0 | 120*^{d}* | 100 [100] | 50 |
| 6 | 2.0 | 10 | 100 [100] | 50 |
| 7 | 1.0 | 120 | 46 [50] | 46 |
| 8 | 1.0 | 30 | 50 [50] | 50 |
| 9 | 1.0 | 10 | 45 [50] | 45 |
| 10 | 0.1 | 120 | 4.9 [5.0] | 49 |
| 11 | 3×0.1 | 3×120 | 15 [15] | 50 |

### I.2 Preparation of Aryldiazonium Chlorides in round bottom flask (batch reaction, GM 2)

In a 250 mL round bottom flask equipped with a balloon for pressure equilibration, nitrogen monoxide (45 ml, ca. 2.0 mmol) was added by syringe to a solution of **1a-1k** (1.00 mmol) in a mixture of water and 1 N HCl (3:1 v/v, 16 ml). After vigorous stirring for 30 minutes at room temperature, the reaction mixture was concentrated under reduced pressure and analyzed by ¹H-NMR spectroscopy. Yields determined by ¹H-NMR using maleic acid as internal standard (after concentration of the reaction mixture in vacuo). The results obtained under these conditions are summarized in the table below.

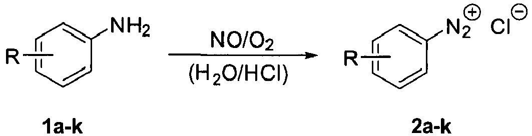

| Entry | **1**: R = | Yield **2** (%) | Side-product |
|---|---|---|---|
| 1 | **1a**: 4-Cl | **2a** (*quant.*) | - |
| 2 | **1b**: 3-Cl | **2b** (*quant.*) | - |
| 3 | **1c**: 2-Cl | **2c** (*quant.*) | - |
| 4 | **1d**: 4-F | **2d** (*quant*.) | - |
| 5 | **1e**: 4-Br | **2e** (88) | **2a** (12) |
| 6 | **1f**: 4-I | **2f** (71) | **2a** (4) |
| 7 | **1g**: 4-Me | **2g** (95) | - |
| 8 | **1h**: 4-OH | **2h** (*quant.*) | - |
| 9 | **1i**: 4-CN | **2i** (91) | - |
| 10 | **1j**: 4-NO₂ | **2j** (*quant.*) | - |
| 11 | **1k**: H | **2k** (19) | diverse |

### I.3 Preparation of Chlorophenyldiazonium Chloride in a 10-liter wet scrubber (GM 3)

A stream of nitrogen dioxide (generated by slow addition of copper powder (0.5 equiv.) to concentrated nitric acid (2.0 equiv.)) in air (0.5, 2, 4 or 6 vol.-% NO₂, total flow: ca. 5 L/min) was passed through a 10-liter wet scrubber (for figure see above) containing 4-chloroaniline (**1a**) (12.8 g, 100 mmol) in a mixture of water and 1 N HCl over a time of 22.4, 67.2 or 269 minutes at room temperature. In the 10-liter wet scrubber, the gas stream got into contact with the scrubber solution while passing in counterstream through a column (length ca. 80 cm, diameter ca. 10 cm) filled with Raschig rings. After the NO₂ generation was finished, the air flow through the scrubber was maintained for another 38 minutes. The content of NO₂ in the outgoing gas stream was measured over the time of NO₂ generation and for some further minutes. The results obtained under these conditions are summarized in the table below.

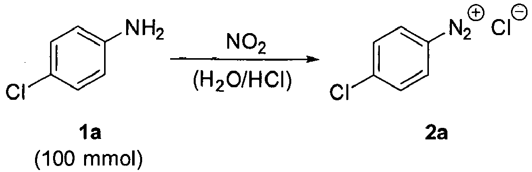

| | NO₂ (mmol) | Addition time^{a} (min) | NO₂ in air (vol.-%) | Solvents 1N HCl/H₂O (L/L) | Diazotization (%)^{b} |
|---|---|---|---|---|---|
| 1 | 200 | 22.4 | 4.00 | 0.8/3.2 | 94 |
| 2 | 300 | 22.4 | 6.00 | 0.8/3.2 | 100 |
| 3 | 300 | 67.2 | 2.00 | 0.8/3.2 | 100 |
| 4 | 300 | 269 | 0.50 | 0.8/3.2 | 100 |
| 5 | 300 | 22.4 | 6.00 | 0.6/2.4^{c} | 100 |
| 6 | 300 | 22.4 | 6.00 | 0.5/3.5 | 100 |
| 7 | 300 | 22.4 | 6.00 | 0.3/3.7 | 100 |
| 8 | 300 | 22.4 | 6.00 | 0.12/3.88 | 96 |

| | | | | | |
|---|---|---|---|---|---|
| ^{a}Time over which NO and NO₂-containing gas stream is passed through the scrubber. ^{b}Yields determined by ¹H-NMR using maleic acid as internal standard (after concentration in vacuo). ^{c}Total amount of solvent reduced to 3 liters. | | | | | |

For the reactions in entries 1-8 the following NOₓ (= NO + NO₂) concentrations were measured in the outgoing gas stream.

| | Addition time (min) | NO₂ in ingoing gas stream (ca. ppm) | NO + NO₂ in outgoing gas stream (max. ppm value) / (measured time span in min) |
|---|---|---|---|
| 1 | 22.4 | 40000 | 190 / (0-30) |
| 2 | 22.4 | 60000 | 130 / (0-30) |
| 3 | 67.2 | 20000 | 145 / (0-70) |
| 4 | 269 | 5000 | 110 / (0-270) |
| 5 | 22.4 | 60000 | 120 / (0-30) |
| 6 | 22.4 | 60000 | 180 / (0-30) |
| 7 | 22.4 | 60000 | 110 / (0-30) |
| 8 | 22.4 | 60000 | 180 / (0-30) |

### II.1 Methods for Biaryl Synthesis

### II.1.1 Method for Biaryl Synthesis with Phenylendiamine 3a

### II.1.1.1 Method for Biaryl Synthesis with Phenylendiamine on 1 mmol scale (GM 4)

For this series of experiments, a partially diazotized mixture containing **1a** and **2a** in a ratio of 41:59 was used. The obtained results demonstrate that radical biaryl synthesis can be performed also with partially diazotized reaction mixtures.

A 16 mL aliquot of the 0.062 M solution of 4-chlorophenyl diazonium chloride **(2a)** (ca. 1 mmol) was added dropwise by syringe pump to a vigorously stirred solution of 1,4-phenylenediamine (**3a**) (2.16 g, 20.0 mmol) in degassed water (10 mL) and hydrochloric acid (7 ml, 3 N HCl) and titanium(III)-chloride (0.5 mmol, 0.5 mL, ca. 1M solution in 3 N HCl) over a period of 7 minutes under argon. After the addition was complete, the mixture was left to stir for 10 more minutes and a solution of sodium hydroxide (4.0 g) and sodium sulfite (4.0 g) in water (40 mL) was added. After extraction with diethyl ether (3×100 mL), the combined organic phases were washed with satd. aqueous sodium chloride and dried over sodium sulfate. Yield determined by internal standard dimethyltherephthalate and 1,3,5-trimethoxybenzene. Concentration in vacuo and purification by column chromatography (*n-*hexane/ethyl acetate 8:1) gave the biphenyl-2,5-diamine (**4a**). The results obtained under these conditions are summarized in the table below.

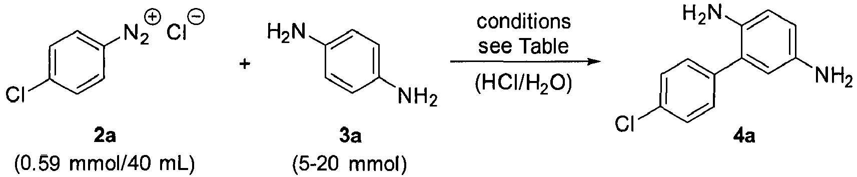

| Entry | Variation from standard conditions described above | Yield **4a** (%) |
|---|---|---|
| 1 | -- | 73 |
| 2 | 1,4-Phenylenediamine **4a** (5 mmol) | 70 |
| 3 | Under air atmosphere | 66 |
| 4 | Absence of TiCl₃ | 69 |
| 5 | TiCl₃ (2.5 mmol) | 64 |
| 6 | Addition of **2a** over 15 min | 69 |
| 7 | Addition of **2a** in one batch | 68 |
| 8 | Reaction at 75°C | 56 |

### II. 1.1.2 Method for Biaryl Synthesis with Phenylendiamine on larger scale (GM 5)

A 400 mL aliquot of the 0.025 M solution of 4-chlorophenyl diazonium chloride (**2a**) (ca. 10 mmol, obtained as described in GM 3, I.3, entries from Table in I.3 correspond to entries in Table II.1.1.2 below) was added by dropping funnel to a vigorously stirred solution of 1,4-phenylenediamine **(3a)** (5.40 g, 50.0 mmol) in degassed water (100 mL), and titanium(III)-chloride (5.0 mmol, 5.0 mL, ca. 1M solution in 3 N HCl) over a period of 7 minutes under argon. In the case that additional hydrochloric acid (25 mL, 75 mmol, 3 N HCl, see entries 1 and 2) was added to **3a** before the addition of **2a,** the amount of water was reduced to 75 mL. For reactions on smaller scales (1, 3, 5 and 7 mmol of **2a**), the amounts of all reagents and solvents were reduced accordingly.

After the addition of **2a** was complete, the mixture was left to stir for 15 more minutes and a solution of sodium hydroxide (20.0 g) and sodium sulfite (20.0 g) in water (400 mL) was added. After extraction with diethylether (3×100 mL), the combined organic phases were washed with saturated aqueous sodium chloride and dried over sodium sulfate. Concentration in vacuo and purification by column chromatography (n-hexane/ethyl acetate 8:1) gave 4'-chlorobiphenyl-2,5-diamine **(4a).** The results obtained under these conditions are summarized in the table below.

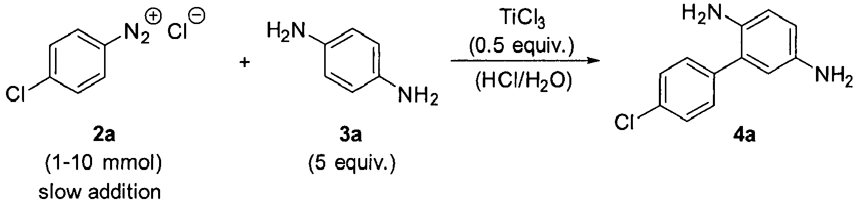

| Entry | HCl added to **3a** (mol) | Yield **4a** from 1mmol **2a** (%) | Yield **4a** from 3mmol **2a** (%) | Yield **4a** from 5mmol **2a (%)** | Yield **4a** from 7mmol **2a** (%) | Yield **4a** from 10mmol **2a** (%) |
|---|---|---|---|---|---|---|
| 1 | 0.75 | - | - | - | - | 29 |
| 2 | 0.75 | - | - | - | - | 32 |
| 3 | 0.0 | - | - | - | - | 45 |
| 4 | 0.0 | 34 | - | 36 | - | 45 |
| 5 | 0.0 | 41 | 43 | 46 | 42 | 47 |
| 6 | 0.0 | - | - | - | - | 56 |
| 7 | 0.0 | 43 | - | 47 | - | 49 |
| 8 | 0.0 | - | - | - | - | 44 |

### II. 1.1.3 Improved work-up procedure for the preparation of 4a (GM 6)

After the addition of **2a** to **3a** and TiCl₃ was complete, the resulting mixture was left to stir for further 15 minutes, and it was then extracted with ethyl acetate (3×100 mL) to remove unpolar side products. Due to the acidic conditions, the biphenyl diamine **4a** as well as unreacted 1,4-phenylenediamine (3a) were protonated and remained in the aqueous phase. Subsequently, a solution of sodium hydroxide (20.0 g) and sodium sulfite (20.0 g) in water (400 mL) was added to the aqueous phase. After extraction with diethyl ether (3×100 mL), the combined organic phases were washed with saturated aqueous sodium chloride and dried over sodium sulfate. Due to the low solubility of phenylenediamine **3a** in diethylether only trace amounts of **3a** were extracted from the aqueous phase. The purity of **4a** reached with this procedure is comparable to the purity previously observed after column chromatography.

### II.1.2. Method for Biaryl Synthesis with 2-Methylfuran (5a) and α-Hydroxymethylfuran (7a) (GM 7)

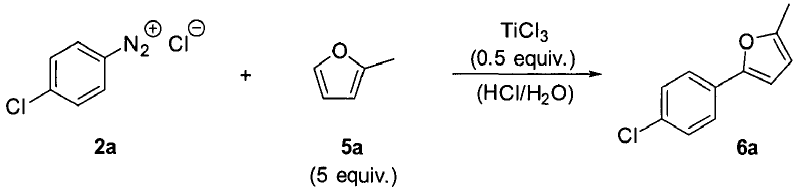

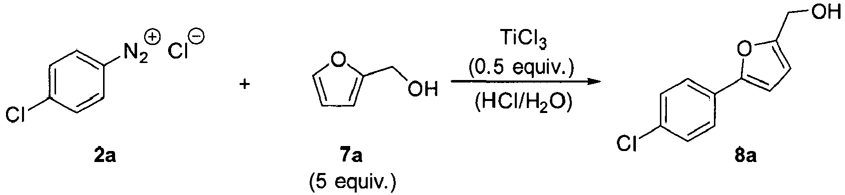

2-Methylfuran (50 mmol, 4.50 mL) or α-Hydroxymethylfuran (50 mmol, 4.34 mL) was added to a vigorously stirred solution of 400 mL aliquot of the 0.025 M solution of 4-chlorophenyl diazonium chloride **(2a)** (ca. 10 mmol, obtained as described in GM 3, I.3, Table entry 6). Subsequently titanium(III)-chloride (5.0 mmol, 5.0 mL, ca. 1 M solution in 3 N HCl) was added and the solution was stirred for 22 minutes. After extraction with dichloromethane (3×200 mL), the combined organic phases were washed with saturated aqueous sodium chloride, dried over sodium sulfate and concentrated in vacuo.
Yields of products **6a** (42%) and **8a** (56%) were determined by ¹H-NMR using internal standards (dimethylterephthalate and 1,3,5-trimethoxybenzene).

### II.2 Method for the preparation of 1-Chloro-4-iodobenzene (9a) (GM 8)

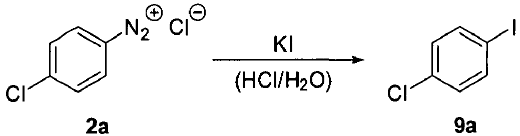

Potassium iodide (30 mmol, 4.98 g) was added to a 400 mL aliquot of the 0.025 M solution of 4-chlorophenyl diazonium chloride **(2a)** (ca. 10 mmol, obtained as described in GM 3, I.3, Table entry 6) and the solution was stirred for 30 minutes at 50° C. After extraction with diethylether (3×200 mL), the combined organic phases were washed with saturated aqueous sodium chloride and dried over sodium sulfate. Concentration in vacuo and purification by column chromatography (diethylether 100%) gave 1-chloro-4-iodobenzene **(9a)** in a yield of 88%.

### II.3 Method for preparation of azo dyes (GM 9)

### II.3.1 Sodium 4-[(4-dimethylamino)phenyldiazenyl]-benzenesulfonate (Methyl orange) (11a) (GM 9)

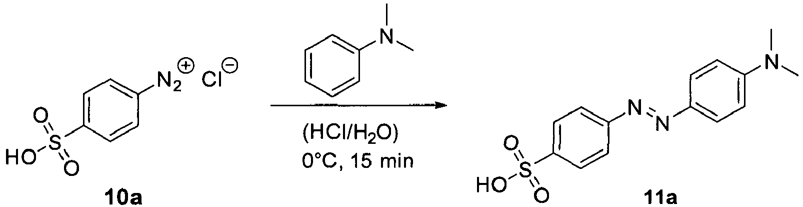

4-Sulfophenyl diazonium chloride (**10a**) (1.00 mmol) is prepared according to GM 2 and added to a solution of *N,N*-dimethylaniline (1.00 mmol, 0.13 ml) in 1 M HCl (1 ml) at 0 °C. After stirring for 15 minutes the solution was neutralized and the precipitate obtained was filtered off, washed with water and concentrated under vacuo. The yield of product **11a** (71 %) was determined by ¹H-NMR spectroscopy using maleic acid as internal standard.

### II.4 Method for preparation of aryl hydrazones (GM 10)

### II.4.1 Ethyl-(E)-2-(2-(4-bromophenyl)hydrazono propanoate (12a) (GM 10)

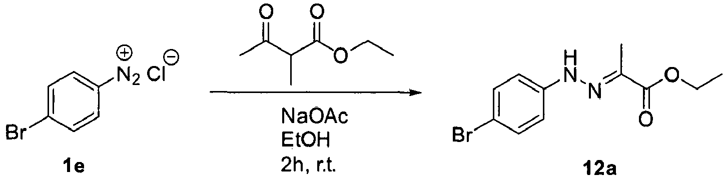

4-Bromo diazonium chloride (**1e**) (1.00 mmol) is prepared according to GM 2 and added to a solution of ethyl-2-methyl-3-oxobutanoate (1.00 mmol, 0.14 mL) and sodium acetate (20.0 mmol, 1.6 g) in ethanol (25 ml). After stirring for two hours the solution was evaporated under reduced pressure. The residue was diluted with ethyl acetate (10 mL), extracted with water (10 mL), dried over sodium sulfate and concentration in vacuo. The yield of product **12a** (30%) was determined by ¹H-NMR spectroscopy using maleic acid as internal standard.

### III. Specific Examples

### III.1. Arenediazonium chlorides

### III.1.1. 4-Chlorobenzenediazonium chloride (2a).

¹H-NMR (360 MHz, DMSO-*d*₆): δ (ppm) = 8.12 (d, *J* = 9.3 Hz, 2 H), 8.76 (d, *J=* 9.3 Hz, 2 H).

### III.1.2. 3-Chlorobenzenediazonium chloride (2b).

¹H-NMR (360 MHz, DMSO-*d*₆): δ (ppm) = 8.01 (t, *J=* 8.3 Hz, 1 H), 8.38 (ddd, *J=* 1.0 Hz, *J =* 2.0 Hz, *J =* 8.3 Hz, 1 H), 8.70 (ddd, *J* = 1.0 Hz, *J* = 2.0 Hz, *J =* 8.3 Hz, 1 H), 8.92 (t, *J* = 2.0 Hz, 1 H).

### III.1.3. 2-Chlorobenzenediazonium chloride (2c).

¹H-NMR (360 MHz, DMSO-*d*₆): δ (ppm) = 7.93-8.00 (m, 1 H), 8.20 (dd, *J* = 1.0 Hz, *J* = 8.3 Hz, 1 H), 8.29 (ddd, *J=* 1.6 Hz, *J* = 7.6 Hz, *J* = 8.3 Hz, 1 H), 8.93 (dd, *J=* 1.6 Hz, *J=* 8.3 Hz, 1 H).

### III.1.4. 4-Fluorobenzenediazonium chloride (2d).

¹H-NMR (360 MHz, DMSO-*d*₆): δ (ppm) = 7.90 (dd, *J* = 8.3 Hz, *J* = 9.4 Hz, 2 H), 8.87 (dd, *J* = 4.5 Hz, *J* = 9.4 Hz, 2 H).

### III.1.5. 4-Bromobenzenediazonium chloride (2e).

¹H-NMR (360 MHz, DMSO-*d*₆): δ (ppm) = 8.26 (d, *J* = 9.2 Hz, 2 H), 8.62 (d, *J* = 9.2 Hz, 2 H).

### III.1.6. 4-Iodobenzenediazonium chloride (2f).

¹H-NMR (360 MHz, DMSO-*d*₆): δ (ppm) = 8.37 (d, *J* = 9.0 Hz, 2 H), 8.43 (d, *J* = 9.0 Hz, 2 H).

### III.1.7. 4-Methylbenzenediazonium chloride (2g).

¹H-NMR (360 MHz, DMSO-*d*₆): δ (ppm) = 2.57 (s, 3 H), 7.80 (d, *J* = 8.3 Hz, 2 H), 8.58 (d, *J* = 8.3 Hz, 2 H).

### III.1.8. 4-Hydroxybenzenediazonium chloride (2h).

¹H-NMR (360 MHz, DMSO-*d*₆): δ (ppm) = 7.27 (d, *J* = 9.3 Hz, 2 H), 8.50 (d, *J* = 9.3 Hz, 2 H).

### III.1.9. 4-Cyanobenzenediazonium chloride (2i).

¹H-NMR (360 MHz, DMSO-*d*₆): δ (ppm) = 8.47 (d, *J* = 9.1 Hz, 2 H), 8.91 (d, *J* = 9.1 Hz, 2 H).

### III.1.10. 4-Nitrobenzenediazonium chloride (2j).

¹H-NMR (360 MHz, DMSO-*d*₆): δ (ppm) = 8.71 (d, *J* = 9.3 Hz, 2 H), 8.96 (d, *J* = 9.3 Hz, 2 H).

### III.1.11. Benzenediazonium chloride (2k).

¹H-NMR (360 MHz, DMSO-*d*₆): δ (ppm) = 7.94-8.02 (m, 2 H), 8.18-8.31 (m, 1 H), 8.63-8.74 (m, 2 H).

### III.2. Biaryl synthesis

### III.2.1. 4'-Chlorobiphenyl-2,5-diamine (4a).

*R*_{f} = 0.5 (EtOAc) [UV]
¹H-NMR (600 MHz, CDCl₃): δ (ppm) = 3.42 (bs, 4 H), 6.52 (d, *J* = 2.5 Hz, 1 H), 6.60 (dd, *J* = 2.5 Hz, *J* = 8.5 Hz, 1 H), 6.64 (d, *J* = 8.5 Hz, 1 H), 7.37-7.41 (m, 4 H). ¹³C-NMR (151 MHz, CDCl₃): δ(ppm) = 116.8 (CH), 117.4 (CH), 117.9 (CH), 127.7 (Cq), 128.9 (2×CH), 130.4 (2×CH), 133.1 (Cq), 135.9 (Cq), 137.9 (Cq), 137.9 (Cq).
HRMS (EI) calculated for C₁₂H₁₁ClN₂ [H⁺]: 219.0684, found: 219.0679.

### III.2.2. 2-(4'-Chlorphenyl)-5-methylfuran (6a).

*R*_{f} = 0.8 (Hexan/EtOAc = 4:1) [UV].
¹H-NMR (600 MHz, CDCl₃): δ (ppm) =2.36 (s, 3 H), 6.06 (m, 1 H), 6.53 (d, *J=* 3.2 Hz, 1 H), 7.32 (d, *J=* 8.0 Hz, 2 H) 7.55 (d, *J* = 8.0 Hz, 2 H).
¹³C-NMR (151 MHz, CDCl₃): δ(ppm) = 13.7 (CH₃), 106.3 (CH), 107.8 (2 × CH), 124.5 (2 × CH), 135.9 (CH), 129.6 (Cq), 132.2 (Cq), 151.2 (Cq), 152.3 (Cq).

### III.2.3. α-Hydroxymethylfuran (7a).

¹H-NMR (600 MHz, CDCl₃): δ (ppm) = 4.66 (s, 2 H), 6.38 (d, *J* = 3.3 Hz, 1 H), 6.58 (d, *J =* 3.3 Hz, 1 H), 7.34 (d, *J =* 8.8 Hz, 2 H) 7.59 (d, *J =* 8.8 Hz, 2 H).

### III.3. Sandmeyer reaction

### III.3.1. 1-Chloro-4-iodobenzene (9a).

¹H-NMR (600 MHz, CDCl₃): δ (ppm) = 7.09 (d, *J* = 8.7 Hz, 2 H), 7.61 (d, *J* = 8.7 Hz, 2 H).

### III.4. Azo coupling

### III.4.1. Sodium 4-[(4-dimethylamino)phenyldiazenyl]-benzenesulfonate (Methyl orange) (11a).

¹H-NMR (600 MHz, D₂O): δ (ppm) = 7.75 (d, *J* = 8.8 Hz, 2 H), 7.59 (dd, *J* = 8.8 Hz, *J=* 14.3 Hz, 4 H), 6.68 (d, *J* = 9.2 Hz, 2 H), 2.86 (s, 6 H).

### III.5. Aryl hydrazones

### III.5.1. Ethyl-(E)-2-(2-(4-bromophenyl)hydrazono propanoate (12a) (mixture of isomers).

¹H-NMR (600 MHz, CDCl₃): δ (ppm) = 7.40 (d, *J* = 8.9 Hz, 2 H, isomer 1), 7.37 (d, *J* = 8.9 Hz, 2 H, isomer 2), 7.10 (d, *J* = 8.9 Hz, 2 H, isomer 1), 7.05 (d, *J* = 8.9 Hz, 2 H, isomer 2), 4.32 (q, *J=* 7.1 Hz, 2 H, isomer 1), 4.27 (q, *J=* 7.1 Hz, 2 H, isomer 2), 2.14 (s, 3 H, isomer 2), 2.10 s (3 H, isomer 1), 1.38 (t, *J=* 7.1 Hz, 3 H, isomer 1), 1.36 (t, *J=* 7.1 Hz, 3 H, isomer 2).

## Claims

1. A process for the preparation of a compound of formula (II) 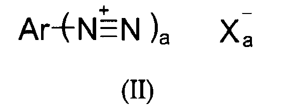 which is **characterized in that** a compound of formula (I) or a salt thereof
Ar(̵NHR')ₐ (I)
is reacted with nitrogen monoxide and/or nitrogen dioxide in the presence of oxygen,
wherein
Ar is an optionally substituted aryl or an optionally substituted heteroaryl group;
R' is hydrogen or a group of formula -CO-R", wherein R" is an optionally substituted alkyl, alkenyl, alkynyl, heteroalkyl, aryl, heteroaryl, cycloalkyl, alkylcycloalkyl, heteroalkylcycloalkyl, heterocycloalkyl, aralkyl or heteroaralkyl group;
X- is an anion; and
a is 1 or 2.

2. The process according to claim 1 for preparing a compound of formula 2, 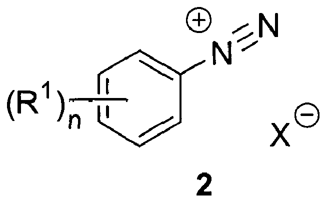 wherein a compound of formula 1 or a salt thereof 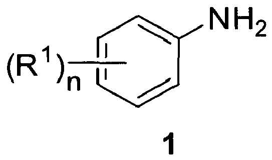 is reacted with nitrogen monoxide and/or nitrogen dioxide in the presence of oxygen,
wherein
n is 0,1,2,3,4 or 5;
R¹ is independently selected from a halogen atom, NO₂, N₃, OH, SH, NH₂, SO₃H, COOH or an optionally substituted alkyl, alkenyl, alkynyl, heteroalkyl, aryl, heteroaryl, cycloalkyl, alkylcycloalkyl, heteroalkylcycloalkyl, heterocycloalkyl, aralkyl or heteroaralkyl group; and
X- is defined as in claim 1.

3. The process according to claim 1 for preparing a compound of formula 4, 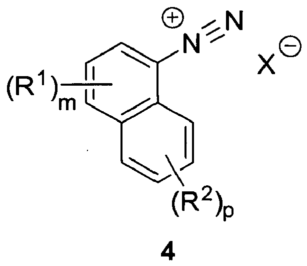 wherein a compound of formula 3 or a salt thereof 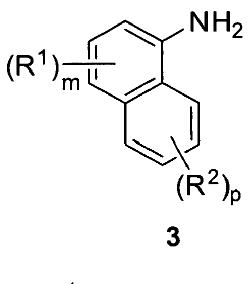 is reacted with nitrogen monoxide and/or nitrogen dioxide in the presence of oxygen,
wherein
m is 0,1,2 or 3;
p is 0,1,2,3 or 4;
R¹ is independently selected from a halogen atom, NO₂, N₃, OH, SH, NH₂, SO₃H, COOH or an optionally substituted alkyl, alkenyl, alkynyl, heteroalkyl, aryl, heteroaryl, cycloalkyl, alkylcycloalkyl, heteroalkylcycloalkyl, heterocycloalkyl, aralkyl or heteroaralkyl group;
R² is independently selected from a halogen atom, NO₂, N₃, OH, SH, NH₂, SO₃H, COOH or an optionally substituted alkyl, alkenyl, alkynyl, heteroalkyl, aryl, heteroaryl, cycloalkyl, alkylcycloalkyl, heteroalkylcycloalkyl, heterocycloalkyl, aralkyl or heteroaralkyl group; and
X- is defined as in claim 1.

4. The process according to claim 1 for preparing a compound of formula 6, 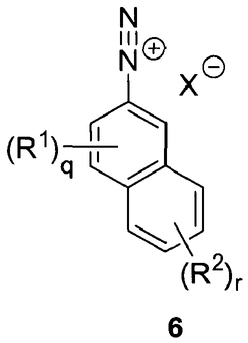 wherein a compound of formula 5 or a salt thereof 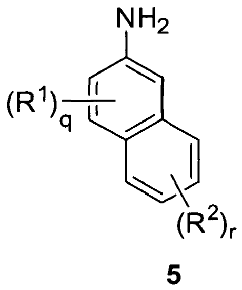 is reacted with nitrogen monoxide and/or nitrogen dioxide in the presence of oxygen,
wherein
q is 0,1,2 or 3;
r is 0,1,2,3 or 4;
R¹ is independently selected from a halogen atom, NO₂, N₃, OH, SH, NH₂, SO₃H, COOH or an optionally substituted alkyl, alkenyl, alkynyl, heteroalkyl, aryl, heteroaryl, cycloalkyl, alkylcycloalkyl, heteroalkylcycloalkyl, heterocycloalkyl, aralkyl or heteroaralkyl group;
R² is independently selected from a halogen atom, NO₂, N₃, OH, SH, NH₂, SO₃H, COOH or an optionally substituted alkyl, alkenyl, alkynyl, heteroalkyl, aryl, heteroaryl, cycloalkyl, alkylcycloalkyl, heteroalkylcycloalkyl, heterocycloalkyl, aralkyl or heteroaralkyl group; and
X- is defined as in claim 1.

5. The process according to claim 1 wherein the compound of formula (I) is selected from the following compounds:

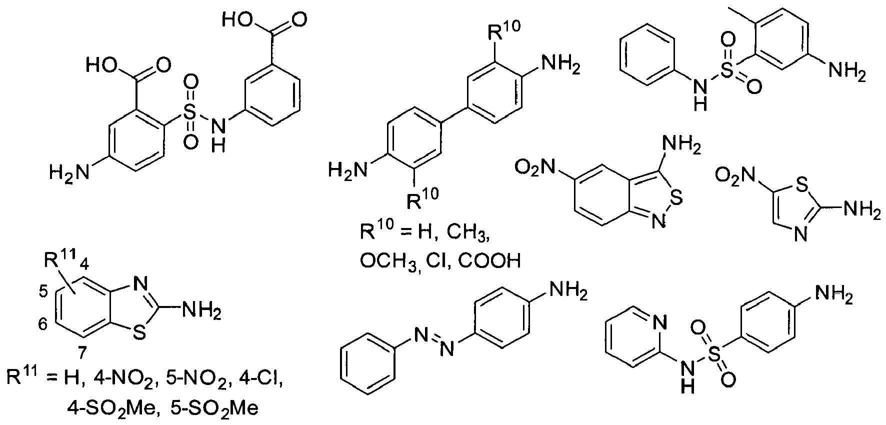

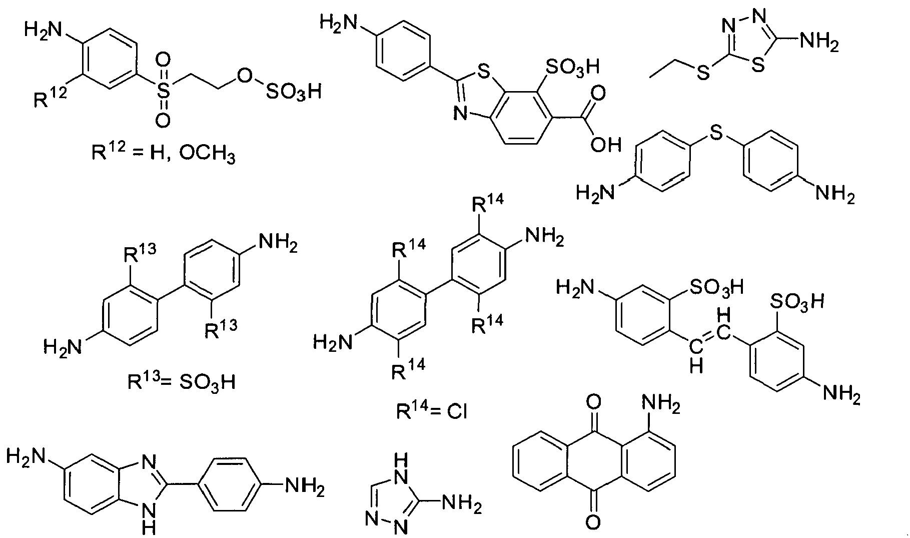

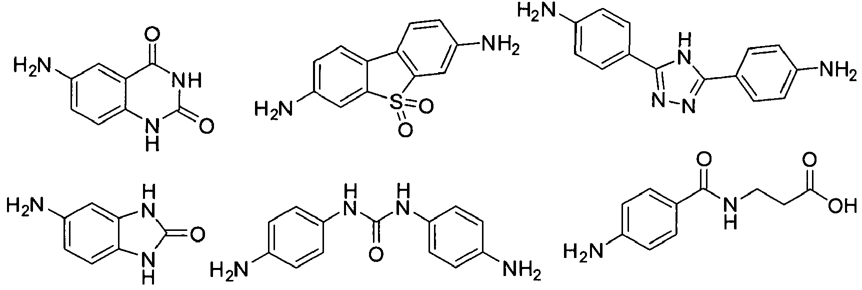

6. The process of claim 1 which further comprises a second step, in which the compound of formula (II) is reacted with a compound of formula Ar'-H to give a compound of formula Ar-N=N-Ar' wherein Ar is defined as in claim 1 and Ar' is an optionally substituted aryl or an optionally substituted heteroaryl group.

7. The process according to any one of the preceeding claims which is carried out in the presence of at least one solvent, preferably in the presence of water or methanol.

8. The process according to any one of the preceeding claims which is carried out in the presence of at least one acid, preferably selected from hydrochloric acid, hydrobromic acid, sulfuric acid, nitric acid, a hydrogensulfate salt, boric acid, tetrafluoroboric acid, methanesulfonic acid, acetic acid and trifluoroacetic acid.

9. The process according to any one of claims 2 to 4 wherein R¹ is independently halogen, hydroxy, amino, alkyl, alkoxy, nitro, cyano, aryl, heteroaryl, NHCOCH₃, arylazo (such as e.g. phenylazo), heteroarylazo, COOH, SO₃H, SO₃M or COOM, wherein M is Li, Na, K, Cs, Mg, Ca, Al or NH₄⁺; especially wherein R¹ is independently halogen, alkyl, alkoxy, hydroxy, amino, nitro, cyano, SO₃H or COOH.

10. The process according to any one of claims 3, 4 or 9, wherein R² is independently halogen, hydroxy, amino, alkyl, alkoxy, nitro, cyano, aryl, heteroaryl, NHCOCH₃, arylazo (such as e.g. phenylazo), heteroarylazo, COOH, SO₃H, SO₃M or COOM, wherein M is Li, Na, K, Cs, Mg, Ca, Al or NH₄⁺; especially wherein R² is independently halogen, alkyl, alkoxy, hydroxy, amino, nitro, cyano, SO₃H or COOH.

11. The process according to any one of the preceeding claims wherein X- is fluoride, chloride, bromide, sulfate, hydrogensulfate, nitrate, nitrite, tetrafluoroborate, acetate, trifluoroacetate, hexafluorophosphate, hexafluoroantimonate, methanesulfonate, trifluoromethanesulfonate, the anion of an aromatic 1,2-dicarboximide or the anion of an aromatic 1,2-disulfonimide, toluene-4-sulfonate or monomethylsulfate; preferably, the anion X- is derived from hydrochloric acid, hydrobromic acid, sulfuric acid, a hydrogensulfate salt, boric acid, tetrafluoroboric acid, methanesulfonic acid, acetic acid, trifluoroacetic acid, nitric acid or nitrous acid; especially preferably from hydrochloric acid or sulfuric acid.

12. The process according to any one of the preceeding claims wherein the nitrogen monoxide and/or nitrogen dioxide as well as the oxygen which are used in the process of the present invention are used in the form of waste gases from industrial processes.

13. The process according to any one of the preceeding claims which is used for the denitrification of waste gases, especially industrially produced waste gases containing nitrogen monoxide and/or nitrogen dioxide.

14. A process for the denitrification of industrial waste gases containing nitrogen monoxide and/or nitrogen dioxide and oxygen which is **characterized in that** the waste gas is brought into contact with an aqueous solution of a compound of formula (I) or a salt thereof
Ar(̵NHR')ₐ (I)
whereby this compound of formula (I) is converted to a compound of formula (II) 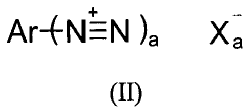 wherein
Ar is an optionally substituted aryl or an optionally substituted heteroaryl group;
R' is hydrogen or a group of formula -CO-R", wherein R" is an optionally substituted alkyl, alkenyl, alkynyl, heteroalkyl, aryl, heteroaryl, cycloalkyl, alkylcycloalkyl, heteroalkylcycloalkyl, heterocycloalkyl, aralkyl or heteroaralkyl group;
X- is an anion; and
ais 1 or2.

15. The process according to claim 14 which is carried out in the presence of at least one acid, preferably selected from hydrochloric acid, hydrobromic acid, sulfuric acid, nitric acid, a hydrogensulfate salt, boric acid, tetrafluoroboric acid, methanesulfonic acid, acetic acid and trifluoroacetic acid.
